(19) Europäisches Patentamt
European Patent Office
Office européen des brevets

(11) EP 3 416 076 A1

# (12) EUROPEAN PATENT APPLICATION

(43) Date of publication:
**19.12.2018 Bulletin 2018/51**

(51) Int Cl.:
***G06F 19/22*** *(2011.01)* ***B82Y 10/00*** *(2011.01)*

(21) Application number: **18177823.4**

(22) Date of filing: **14.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.06.2017 RU 2017120555**

(71) Applicants:
- **Landigrad, Limited Liability Company**
  **170026 Tver (RU)**
- **Smirnov, Sergey Nikolayevich**
  **170033 Tver (RU)**

(72) Inventor: **SMIRNOV, Sergey Nikolayevich**
**170033 Tver (RU)**

(74) Representative: **Cabinet Chaillot**
**16/20, avenue de l'Agent Sarre**
**B.P. 74**
**92703 Colombes Cedex (FR)**

## (54) METHODS OF CODING AND DECODING INFORMATION

(57) The inventions fall into the category of means of coding/ decoding of all types of information (text file, images, sound files) using DNA, RNA and amino acid sequences. A coding method was suggested according to which a data array to be coded is divided into chunks, each of which is assigned at least one element of the molecular genetic system; the set of elements in the system is supplemented by relevant indexing information consisting of i data bits and k check bits. Each piece of i+k bit data shall be written as a multiplet of the molecular genetic system consisting of the n number of nitrogenous bases or corresponding amino acids. Besides the information to be coded, one shall also write the value n and the information about the selected method of transforming the matrix into a sequence and the reading order. A decoding method was suggested according to which the machine-readable sequence is divided into chunks of information to be decoded, which includes combinations of i data bits and k check bits, and assigned at least one multiplet of the molecular genetic system consisting of the n number of nitrogenous bases or corresponding amino acids. The molecular genetic system used for the coding/ decoding consists of a base representing a four-nitrogenous-base matrix $\begin{bmatrix} G & C \\ A & T/U \end{bmatrix}$; the nitrogenous bases are grouped according to the number of hydrogen bonds and the number of condensed rings contained in the molecular structures of nitrogenous bases; the matrix is formed in advance by raising tensors of each previous-generation matrix to the second power multiple times in order to obtain a next-generation matrix. The system binary indices that form the i data bits correspond to the selected characteristic of the nitrogenous bases. As a result, the effectiveness of antinoise coding and decoding is enhanced due to the increase in the volume of transmitted and received information and due to the decrease in the number of elements used for coding/ decoding. 2 independent claims, 18 dependent claims, 17 illustrations.

EP 3 416 076 A1

7 6 5 4

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 |
| | 1 | 1 | 0 | 0 | 1 | 1 | 0 | 0 | 1 | 1 | 0 | 0 | 1 | 1 | 0 | 0 | 1 | 1 | 0 | 0 | 1 | 1 | 0 | 0 | 1 | 1 | 0 | 0 | 1 | 1 | 0 | 0 |
| | 1 | 1 | 1 | 1 | 0 | 0 | 0 | 0 | 1 | 1 | 1 | 1 | 0 | 0 | 0 | 0 | 1 | 1 | 1 | 1 | 0 | 0 | 0 | 0 | 1 | 1 | 1 | 1 | 0 | 0 | 0 | 0 |
| | 1 | 1 | 1 | 1 | 1 | 1 | 1 | 1 | 0 | 0 | 0 | 0 | 0 | 0 | 0 | 0 | 1 | 1 | 1 | 1 | 1 | 1 | 1 | 1 | 0 | 0 | 0 | 0 | 0 | 0 | 0 | 0 |
| | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 |
| | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| | 11 | 10 | 11 | 10 | 11 | 10 | 11 | 10 | 01 | 00 | 01 | 00 | 01 | 00 | 01 | 00 | 11 | 10 | 11 | 10 | 11 | 10 | 11 | 10 | 01 | 00 | 01 | 00 | 01 | 00 | 01 | 00 |
| 3' | 11 | 11 | 10 | 10 | 11 | 11 | 10 | 10 | 11 | 11 | 10 | 10 | 11 | 11 | 10 | 10 | 01 | 01 | 00 | 00 | 01 | 01 | 00 | 00 | 01 | 01 | 00 | 00 | 01 | 01 | 00 | 00 |
| | 11 | 11 | 11 | 11 | 10 | 10 | 10 | 10 | 11 | 11 | 11 | 11 | 10 | 10 | 10 | 10 | 11 | 11 | 11 | 11 | 10 | 10 | 10 | 10 | 11 | 11 | 11 | 11 | 10 | 10 | 10 | 10 |
| Z | G | C | G | C | G | C | G | C | A | T/U | A | T/U | A | T/U | A | T/U | G | C | G | C | G | C | G | C | A | T/U | A | T/U | A | T/U | A | T/U |
| Y | G | G | C | C | G | G | C | C | G | G | C | C | G | G | C | C | A | A | T/U | T/U | A | A | T/U | T/U | A | A | T/U | T/U | A | A | T/U | T/U |
| X | G | G | G | G | C | C | C | C | G | G | G | G | C | C | C | C | G | G | G | G | C | C | C | C | G | G | G | G | C | C | C | C |
| 5' | Gly (G) | Gly (G) | Ala (A) | Ala (A) | Arg (R) | Arg (R) | Pro (P) | Pro (P) | Gly (G) | Gly (G) | Ala (A) | Ala (A) | Arg (R) | Arg (R) | Pro (P) | Pro (P) | Glu (E) | Asp (D) | Val (V) | Val (V) | Gln (Q) | His (H) | Leu (L) | Leu (L) | Glu (E) | Asp (D) | Val (V) | Val (V) | Gln (Q) | His (H) | Leu (L) | Leu (L) |

3 2 1 0

| | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|
| 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 |
| 1 | 1 | 0 | 0 | 1 | 1 | 0 | 0 | 1 | 1 | 0 | 0 | 1 | 1 | 0 | 0 | 1 | 1 | 0 | 0 | 1 | 1 | 0 | 0 | 1 | 1 | 0 | 0 | 1 | 1 | 0 | 0 |
| 1 | 1 | 1 | 1 | 0 | 0 | 0 | 0 | 1 | 1 | 1 | 1 | 0 | 0 | 0 | 0 | 1 | 1 | 1 | 1 | 0 | 0 | 0 | 0 | 1 | 1 | 1 | 1 | 0 | 0 | 0 | 0 |
| 1 | 1 | 1 | 1 | 1 | 1 | 1 | 1 | 0 | 0 | 0 | 0 | 0 | 0 | 0 | 0 | 1 | 1 | 1 | 1 | 1 | 1 | 1 | 1 | 0 | 0 | 0 | 0 | 0 | 0 | 0 | 0 |
| 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| 11 | 10 | 11 | 10 | 11 | 10 | 11 | 10 | 01 | 00 | 01 | 00 | 01 | 00 | 01 | 00 | 11 | 10 | 11 | 10 | 11 | 10 | 11 | 10 | 01 | 00 | 01 | 00 | 01 | 00 | 01 | 00 |
| 11 | 11 | 10 | 10 | 11 | 11 | 10 | 10 | 11 | 11 | 10 | 10 | 11 | 11 | 10 | 10 | 01 | 01 | 00 | 00 | 01 | 01 | 00 | 00 | 01 | 01 | 00 | 00 | 01 | 01 | 00 | 00 |
| 01 | 01 | 01 | 01 | 00 | 00 | 00 | 00 | 01 | 01 | 01 | 01 | 00 | 00 | 00 | 00 | 01 | 01 | 01 | 01 | 00 | 00 | 00 | 00 | 01 | 01 | 01 | 01 | 00 | 00 | 00 | 00 |
| G | C | G | C | G | C | G | C | A | T/U | A | T/U | A | T/U | A | T/U | G | C | G | C | G | C | G | C | A | T/U | A | T/U | A | T/U | A | T/U |
| G | G | C | C | G | G | C | C | G | G | C | C | G | G | C | C | A | A | T/U | T/U | A | A | T/U | T/U | A | A | T/U | T/U | A | A | T/U | T/U |
| A | A | A | A | T/U | T/U | T/U | T/U | A | A | A | A | T/U | T/U | T/U | T/U | A | A | A | A | T/U | T/U | T/U | T/U | A | A | A | A | T/U | T/U | T/U | T/U |
| Arg (R) | Ser (S) | Thr (T) | Thr (T) | Trp (W) | Cys (C) | Ser (S) | Ser (S) | Arg (R) | Ser (S) | Thr (T) | Thr (T) | Opal | Cys (C) | Ser (S) | Ser (S) | Lys (K) | Asn (N) | Met (M) | Ile (I) | Amber | Tyr ( Y) | Leu (L) | Phe (F) | Lys (K) | Asn (N) | Ile (I) | Ile (I) | Ochre | Tyr ( Y) | Leu (L) | Phe (F) |

Fig. 1

## Description

FIELD OF THE INVENTION

The present inventions relate to the spheres of biochemistry, bio-pharmacology, bio-technology, genetic engineering, and hands-on programming of embedded physical and biochemical processes and technologies, namely the methods of coding all types of information (text files, images, sound files) using DNA, RNA and amino acid sequences for its further storage, processing, and transmission/ receipt.

BACKGROUND OF INVENTION

There is a ***conventional*** DNA-based method of coding text information (patent # US 6,312,911, IPC C12Q1/68, G06N3/12, 2001) which describes the process of coding text messages into DNA sequences and subsequent message extraction using the molecular genetic system, each element of which consists of three nitrogenous bases of the DNA and represents an alphanumeric symbol. Since the DNA has 4 bases (A - adenine, T - thymine, C - cytosine, G - guanine), the maximal triplet representation of the known molecular genetic system will consist of 64 unique symbols, which number is equal to the number of combinations made from the four nitrogenous bases.

There is a ***conventional*** method of coding any type of information (text files, images or sound files) described in the patent № US 2005/0053968 (IPC G06F 19/00, C12Q 1/68, G06N 3/12, G01N 33/48, G11B 20/00, G01N 33/50, G11C 13/02, 2005), which uses the molecular genetic system consisting of various combinations of the four nitrogenous bases of the DNA (G, A, C, T), where each combination represents a unique symbol. The ***conventional*** method is used to create a synthetic DNA molecule that will include the digital information and the encryption key. The synthetic DNA is placed into the DNA carrier for purpose of storage. In cases where the amount of DNA is too big, the information can be divided into several chunks. The method described in the patent can restore divided chunks of DNA by means of comparing the primer of one of the chunks with the end primers of one of the possible subsequent chunks.

The application of the ***conventional*** methods is limited since they cannot be used for effective coding of large volumes of information and have low noise-immunity.

The prior art provides data on ***conventional*** redundant coding of digital information with a noise-immune code, in which k check bits are added to data bits in order to increase the noise-immunity; thanks to the check bits one can identify and/ or correct errors that may appear in the decoder due to the communication channel. ***Conventional*** method of noise-immune coding that uses check bits was described, e.g., in the patent No. RU 2408979 (IPC H03M 13/19, 2011).

The method that is the closest to the one stated in the claim is the method of coding information, according to which the data array to be coded is divided into discrete components (symbols), and each selected symbol is associated with at least one triplet, then a unique DNA sequence is constructed, which is split into a set of overlapping chunks of DNA. Then the set of DNA chunks is supplemented by relevant indexing information consisting of i data bits, through which the information being coded is represented as a machine-readable sequence in binary form. (Patent № US 61/654,295, IPC G06F 19/00, 2013).

The drawback of this method is its comparatively high redundancy which takes place due to the fact that quite a substantial number of triplets is required for the coding, which can lead to a decrease in the efficiency of coding and errors in coding; another drawback is the low information capacity of each of the nitrogenous bases which is about 1.83 bits.

SUMMARY OF THE INVENTION

The capacity of the DNA to hold vast amounts of information is currently one of the properties of the DNA which is being researched in order for the DNA to be used as a data carrier. DNA molecules ensure the high density of the information being stored; they are durable and can store information for hundreds of years if kept in proper conditions (i.e., cool dry and dark areas). From the perspective of the theory of noise immunity in digital communication and the transmission of discrete signals, one can say that genetic information is inherently immune to noise. However, the problem that arises when using the known methods of coding and decoding information remains unsolved, which means that the original information gets distorted during the coding process due to various external factors, such as defective DNA synthesis, degradation of DNA molecules which occurs with time and errors in the sequence structure. That is why, despite the previous developments, systematic research and classification of all specific interactions between the sequences of nitrogenous bases are still needed in order to define the conditions of effective, fast and error-free coding of vast volumes of information.

In developing these inventions, it was attempted to solve the problem of keeping large arrays of information without data losses when using the minimal number of elements of the physical storage medium.

As a result, if the suggested solution is implemented, the effectiveness of noise-immune coding and decoding will be enhanced due to the increase in the volume of transmitted and received information and due to the decrease in the number of elements used for the coding and decoding.

Provided is a coding method, wherein a data array is divided into logically complete chunks each of which is assigned at least one element of the molecular genetic system used for the coding; the set of the elements in the system is supplemented by relevant indexing information consisting of i data bits; each code combination of data bits is supplemented by a combination of k check bits defined based on the combination of i data bits; whereas, ***according to the invention,*** each piece of i+k bit data in binary form is written as a multiplet that consists of the n number of nitrogenous bases or corresponding amino acids and be an element of the molecular genetic system, consisting of a base representing a four-nitrogenous-base matrix $\begin{bmatrix} G & C \\ A & T/U \end{bmatrix}$; the nitrogenous bases are grouped according to the number of hydrogen bonds and the number of condensed rings contained in the molecular structures of nitrogenous bases; the matrix is preformed by raising tensors of each previous-generation matrix to the second power multiple times in order to obtain a next-generation matrix. The system binary indices that form the i data bits correspond to the selected characteristic of the nitrogenous bases and write the information being coded, the n value that defines the size of the matrix used for coding, and the information about the selected method of transforming the matrix into a sequence and the reading order.

Whereas, ***according to the invention,*** the information to be coded is lined up as a machine-readable sequence in binary form including binary indices for each multiplet.

Whereas, ***according to the invention,*** the information to be coded is lined up as a sequence of nitrogenous bases.

Whereas, ***according to the invention,*** each code combination of i data bits is supplemented by a combination of m control bits defined depending on the combination of i and k bits;

Whereas, ***according to the invention,*** judging by the position of each multiplet in the matrix of the molecular genetic system, one shall identify whether the corresponding logically complete chunk has a recessive or dominant trait.

Whereas, ***according to the invention,*** the sequence of nitrogenous bases is split into logically complete chunks, in which the information about the beginning and the end of the chunk of information is also coded.

Whereas, ***according to the invention,*** the information to be coded is lined up as a sequence of nitrogenous bases in order to be coded on the DNA level.

Whereas, ***according to the invention,*** the information to be coded is lined up as a sequence of nitrogenous bases in order to be coded on the RNA level.

Whereas, ***according to the invention,*** the information to be coded is lined up as a sequence of amino acids.

Whereas, ***according to the invention,*** the information to be coded is lined up as a sequence of nitrogenous bases to be coded on the protein level.

Whereas, ***according to the invention,*** the molecular genetic system has a linear matrix representation.

Whereas, ***according to the invention,*** the molecular genetic system has a square matrix representation.

Whereas, ***according to the invention,*** the molecular genetic system has a rectangular matrix representation.

Whereas, ***according to the invention,*** the molecular genetic system has a circular matrix representation.

Whereas, ***according to the invention,*** the molecular genetic system has a volume representation.

Whereas, ***according to the invention,*** the molecular genetic system has a tree structure representation.

Whereas, ***according to the invention,*** the binary indices of the molecular genetic system correspond to the selected characteristic of nitrogenous bases for the DNA.

Whereas, ***according to the invention,*** the binary indices of the molecular genetic system correspond to the selected characteristic of nitrogenous bases for the RNA.

Whereas, ***according to the invention,*** the binary indices of the molecular genetic system correspond to the selected characteristic of nitrogenous bases for amino acids.

Whereas, ***according to the invention,*** the binary indices of the molecular genetic system correspond to the selected characteristic of nitrogenous bases forming recessive and dominant traits on the genetic level.

Whereas, ***according to the invention,*** the synthetic DNA formed based on the obtained sequence of amino acids and containing the information to be coded is put in a storage medium.

Whereas, ***according to the invention,*** the synthetic DNA formed based on the obtained sequence of nitrogenous bases and containing the information to be coded is put in medium for logical and mathematical calculations.

Provided is a decoding method, wherein a machine-readable sequence is divided into chunks consisting of logically complete fragments of information to be decoded; the chunks include combinations of i data bits and k check bits, and each logically complete fragment is assigned at least one multiplet - which is an element of the molecular genetic system used for decoding - from the n number of nitrogenous bases or corresponding amino acids; whereas, ***according to the invention,*** the molecular genetic system consists of a base representing a four-nitrogenous-base

matrix $\begin{bmatrix} G & C \\ A & T/U \end{bmatrix}$; the nitrogenous bases are grouped according to the number of hydrogen bonds and the number of condensed rings contained in the molecular structures of nitrogenous bases; the matrix is preformed by raising tensors of each previous-generation matrix to the second power multiple times in order to obtain a next-generation matrix. The system binary indices that form the i data bits correspond to the selected characteristic of the nitrogenous bases, and the indices that form the k check bits are defined based on the combinations of i data bits.

Whereas, ***according to the invention,*** the discrete parts of the machine-readable sequence, consisting of logically complete chunks of information to be decoded, include combinations of m control bits; the indices forming the m control bits are defined based on the combinations of i data bits;

Whereas, ***according to the invention,*** on the assumption of the position of each multiplet in the matrix of the molecular genetic system, one shall identify whether the corresponding logically complete chunk has a recessive or dominant trait.

Whereas, ***according to the invention,*** the molecular genetic system has a linear matrix representation.

Whereas, ***according to the invention,*** the molecular genetic system has a square matrix representation.

Whereas, ***according to the invention,*** the molecular genetic system has a rectangular matrix representation.

Whereas, ***according to the invention,*** the molecular genetic system has a circular matrix representation.

Whereas, ***according to the invention,*** the molecular genetic system has a volume representation.

Whereas, ***according to the invention,*** the molecular genetic system has a tree structure representation.

Whereas, ***according to the invention,*** the binary indices of the molecular genetic system correspond to the selected characteristic of nitrogenous bases for the DNA.

Whereas, ***according to the invention,*** the binary indices of the molecular genetic system correspond to the selected characteristic of nitrogenous bases for the RNA.

Whereas, ***according to the invention,*** the binary indices of the molecular genetic system correspond to the selected characteristic of nitrogenous bases for amino acids.

Whereas, ***according to the invention,*** the binary indices of the molecular genetic system correspond to the selected characteristic of nitrogenous bases forming recessive and dominant traits on the genetic level.

In molecular biology, the central dogma is a flow of information from DNA to RNA and from nucleic acids to protein. The transition of genetic information from DNA to RNA and from RNA to amino acids forming protein complexes is standard for all of the organisms that consist of cells and is the basis of the biosynthesis of macromolecules. DNA, RNA, amino acids and proteins are linear polymers, which means that each monomer in their structure is linked up, as a rule, to two other monomers. The sequence of monomers encodes the information, whose transition is described by the central dogma.

The reproduction of DNA molecules and synthesis of RNA molecules is carried out in such a way where the DNA strand serves as a matrix (template) for the construction of a daughter molecule (template-based synthesis). This method ensures that hereditary information is copied and implemented in the amino acid (protein) synthesis. The flow of information includes three types of template-based syntheses: DNA synthesis - replication; RNA synthesis - transcription; and protein synthesis - translation. Besides, there are a template-based synthesis that corrects errors in the structure of DNA (RNA) and a variation of limited replication (SOS-reparation) that restores the original structure of DNA (RNA). The template nature of the nucleic acid and protein synthesis ensures the high accuracy of data reproduction.

Currently, researching the principles of genetic code noise-immunity is of great importance for the technical development, since it can help us to solve the problem of providing information systems with noise-immunity. If information is coded using nitrogenous bases of DNA (RNA) or amino acids, it will represent a very complex binary combination that is defined through the arrangement of multiplets in the system. Decoding information represented in such a way will require an elaborate mathematical approach. The hierarchical system used for coding and decoding allows us to define the stability of each multiplet, its general molecular structure and many other parameters.

The molecules of proteins, nucleic acids (DNA, RNA) and polysaccharides that build up tissues, organs, the intracellular structure (cytoskeleton) and the extracellular matrix, membrane channels, receptors and molecular machines intended for the synthesis, packing and disposal of proteins and nucleic acids relate to biological nanoobjects. The size of protein molecules can be from 1 to 1,000 nm. The diameter of the helix of DNA is 2 nm and its length can reach up to several centimeters. Protein complexes that form the fibers of the cytoskeleton are 7-25 nm thick and are up to several microns long. This feature makes it possible to keep large volumes of information using relatively small amounts of physical storage medium.

The set of the four nitrogenous bases is usually considered to be the elementary alphabet of the genetic code. The genetic information transferred by the hereditary molecules (DNA and RNA) defines the initial structure of proteins of a living organism. Each protein that is being coded represents a strand of 20-22 types of amino acids. The sequence of amino acids in the protein strand is defined by the sequence of triplets (three-letter "words"). A triplet (or a codon) is a combination of three nitrogenous bases located in a sequence on a strand of DNA (or RNA).

Computers usually store information in the form of matrices and process it using matrices of 27 unitary transformations, primarily standardized Hadamard matrices that act as logical units performing various steps after receiving various conditions.

The suggested inventions will allow us to discover structural characteristics of the genetic code based on the mathematics of matrices; the genetic code will be structured as the suggested molecular genetic system representing a hierarchical system of the elements of the genetic code. Whereas, patterns in the suggested system, which can be observed through various characteristics of the nitrogenous bases for DNA, RNA and amino acids, ensure the noise-immunity of the suggested system judging from the viewpoint of the matrix and mathematical methods of the theory of discrete signals and the digital field. Moreover, these patterns can also be observed at the lower (atomic and subatomic) and upper (protein, genetic) levels. Besides, this approach to structuring does not feature the necessary distinction between the DNA and RNA levels. In addition, structuring can be carried out using both major and minor nitrogenous bases.

In addition, one can observe a harmonious and well-adjustable fractal connection in the molecular genetic system used for coding/ decoding on the following levels:

DNA - RNA - Amino acids - Proteins - Genetic traits

Whereas, one can observe clear mathematical relationships on each structural level of the molecular genetic system, when counting the atomic and subatomic components of the nitrogenous bases.

Whereas, it is possible to conduct a color analysis of the molecular genetic system on the structural level, using a black and white chart developed based on the indexing information (1 - black, 0 - white) and color charts, i.e. a fractal color chart and a chart based on the length of the waves of the visible spectrum.

Whereas, apart from the square and circular charts of the molecular genetic system, a linear representation, a rectangular representation, a volume (cubical) representation and a tree structure representation are used.

## BRIEF DESCRIPTION OF THE DRAWINGS

The disclosure will be readily understood by the following detailed description in conjunction with the accompanying drawings, wherein like reference numerals designate like structural elements, and in which:

Fig. 1 shows the linear matrix representation of the matrix used for the coding, which is formed by 64 triplets, for DNA, RNA and amino acids with binary indexing information assigned to each element of the matrix;
Fig. 2 shows the square matrix representation of the triplet matrix for DNA, RNA and amino acids;
Fig. 3 shows the rectangular matrix representation of the triplet matrix for DNA, RNA and amino acids;
Fig. 4 shows the circular matrix representation of the triplet matrix for DNA, RNA and amino acids;
Fig. 5, 5A show the volume (cubical) representation of the triplet matrix for DNA/ RNA;
Fig. 6, 6A show the tree structure matrix representation of the triplet matrix representation for DNA;
Fig. 7 shows the alteration in the number of hydrogen bonds in the linear representation of the triplet matrix for DNA;
Fig. 8 shows the alteration in the number of hydrogen bonds in the rectangular representation of the triplet matrix for DNA/ RNA;
Fig. 9 shows the alteration in the total number of the atoms of carbon (C), hydrogen (H), nitrogen (N), and oxygen (O) for each nitrogenous base in the linear representation of the triplet matrix for DNA;
Fig. 10 shows the alteration in the total number of the atoms of carbon (C) for each nitrogenous base of DNA in the linear representation of the triplet matrix;
Fig. 11 shows the alteration in the total number of the atoms of carbon (C) for each nitrogenous base of RNA in the linear representation of the triplet matrix;
Fig. 12 shows the total number of the atoms of oxygen (O) for each nitrogenous base of DNA in the linear representation of the triplet matrix;
Fig. 13 shows the total number of the atoms of oxygen (O) for each nitrogenous base of RNA in the linear representation of the triplet matrix;
Fig. 14 shows the comparative analysis of the major and minor nitrogenous bases for DNA and RNA;
Fig. 15 shows the table of relationships between information (variable) and reference (resulting) values of the logical operation obtained through adding under the module 2 in the form of 64 triplets of DNA (RNA);
Fig. 16 shows the table of relationships between the resulting vectors and their triplet interpretation;
Fig. 17 shows the alphabet completed based on the molecular genetic system of triplets.

## DETAILED DESCRIPTION OF THE INVENTION

In practice, one can come across variations of digital, text, symbol, graphic and mixed information. The purpose of the inventions is to transform any type of information into a sequence of code combinations consisting of i data bits; each code combination is supplemented by data bits in order to provide an opportunity to restore the information using the code combinations of k check bits, whose combination is defined based on the combinations of the i data bits. Whereas, the combination of the k check bits is selected in accordance with numerical representations that either coincide with the complete orthogonal system of Walsh functions applied in the noise-immune coding for discrete signal processing, or have a constant value.

The array of information to be coded is divided into logically complete chunks (a series of symbols, images, sounds or biological sequences at the genetic and somatic levels), each of which is associated with at least one element of the molecular genetic system consisting of the n number of nitrogenous bases. The set of elements in the system is supplemented by indexing information consisting of i data bits; the indexing information corresponds to the selected characteristic of the nitrogenous bases for DNA, RNA or amino acids. Based on the indexing information, a code check combination consisting of k check bits is formed, e.g., this can be a combination of the values of the Boolean function of adding variables under the module 2, whose numerical representations coincide with the complete orthogonal system of Walsh functions [I.V. Agafonova "Cryptographic Properties of NonLinear Boolean Functions," 2007 http://dha.spb.ru/PDF/cryptoBOOLEAN.pdf]. For instance, the number of corresponding nitrogenous bases for one discrete element of the information to be coded may be equal to 6 (X, Y, Z is the code combination of the element and X'Y'Z' is the code check combination). By means of Z-coding, the information being coded is represented as a machine-readable sequence in binary (discrete) form or as a unique nucleotide sequence made of the n number of multiplets. The n number and the information about the selected method of transforming the matrix into a sequence is recorded together with the information being coded. Thanks to the fractal nature of the molecular genetic system suggested for coding, the multiplet sequence may be made up for RNA, DNA, amino acids and proteins.

DNA and RNA include nucleotides that consist of sugar, a phosphate group and nitrogenous bases: cytosine (C), adenine (A), guanine (G), thymine (T) for DNA and uracil (U) for RNA. Whereas, the nitrogenous bases represent specific structures with special biochemical properties. Since the structure of the sugar and phosphate backbone remains the same, it is the characteristics of the five major nitrogenous bases that define the position of the nucleotides, when the basis of the molecular genetic system of the elements is being constructed; the system is made in the form of a rectangular matrix $F^1$ of the size 2x2. $F^1 = \begin{bmatrix} G & C \\ A & T/U \end{bmatrix},$ where T/U means the application in the construction of the system of thymine (for DNA) or uracil (for RNA).

The basis made of four nitrogenous bases for DNA and four nitrogenous bases for RNA is the medium of a content-rich symmetrical system of distinctive and uniting features that splits the four nitrogenous bases into equivalent pairs based on one of these features. For DNA the basis ($F^1$) is made up with thymine (T), and for the RNA the basis is made up with uracil (U).

The term "molecular genetic system" used in this context means a complex of structures and mechanisms of transition of hereditary information (genetic material) characteristic of this specific type [Arefyev, V.A., Lisovenko, L.A. English-Russian Dictionary of Genetic Terms 1995, 407 p.]. Whereas, the system is an object, whose integrity is provided by a cluster of connections and relations between groups of elements united by structures expanded in the space and time.

The arguments in support of this structure are as follows:

The double helix of the usual DNA consists of two mutually intertwined polynucleotide strands, whose nitrogenous bases are linked up through hydrogen bonds in pairs. The rigidity of the three-dimensional configuration of the DNA is primarily ensured due to the great number of hydrogen bonds between the opposite bases of the strands, which means that the adenine (A) of one of the strands is always opposite to the thymine (T) of the other one, or than guanine (G) is opposite to cytosine (C), which makes one strand of DNA complementary (supplementary) to another one based on the positions of the nitrogenous bases. The adenine (A) of one strands is connected to the thymine (T) of the other one, and guanine (G) is connected to cytosine (C). RNA represents a single-strand molecule structured just like one of the DNA strands. The RNA nucleotides are very similar to those of DNA, although they are not identical. Three of the nitrogenous bases are exactly the same as in the DNA: adenine (A), guanine (G) and cytosine (C). However, instead of thymine (T), the RNA contains another nitrogenous base similar to thymine in structure which is uracil (U).

The nitrogenous bases are horizontally arranged in the basis ($F^1$) presented here depending on the number of their hydrogen bonds: the first line of the matrix features a complementary pair with a strong hydrogen bond G - C: it has three hydrogen bonds; the second line features a complementary pair with a weak hydrogen bond A - T (for DNA)/ U (for RNA): it has two hydrogen bonds.

Secondly, a feature that is specific of nitrogenous bases is the fact that they are subdivided into two types: purine bases that include adenine (A) and guatnine (G) and pyrimidine bases that include cytosine (C), thymine (T) and uracil (U). The molecules of purine and pyrimidine bases are based on the two aromatic heterocyclic compounds - pyramidine and purine. The molecule of purine consists of two condensed rings, and the molecule of pyramidine consists of one condensed ring. Therefore, the nitrogenous bases in the basis ($F^1$) presented here are vertically arranged depending on the number of their condensed rings: the first column of the matrix features purine bases that have a greater number of condensed rings in their molecular structure, and the second column features pyramidine bases that have a fewer number of rings.

Therefore, we obtain a basis ($F^1$) of a system of elements, in which nitrogenous bases are divided in pairs according to their primary characteristics horizontally - with the first line featuring elements with three hydrogen bonds, and the second line featuring elements with two hydrogen bonds - and vertically - with the first column featuring purine bases and the second column featuring pyramidine bases.

In this subdivision into equivalent pairs based on a specific feature, each nitrogenous base can be supplemented by a binary opposition index: e.g., by one when the feature is very pronounced or by zero when it is not very pronounced. In case of applying the subdivision into binary indices based on the biochemical characteristics of nitrogenous bases, the elementary basis ($F^1$) is illustrated as follows:

$$F^1 = \begin{bmatrix} G\ 11 & C\ 10 \\ A\ 01 & T/U\ 00 \end{bmatrix}$$

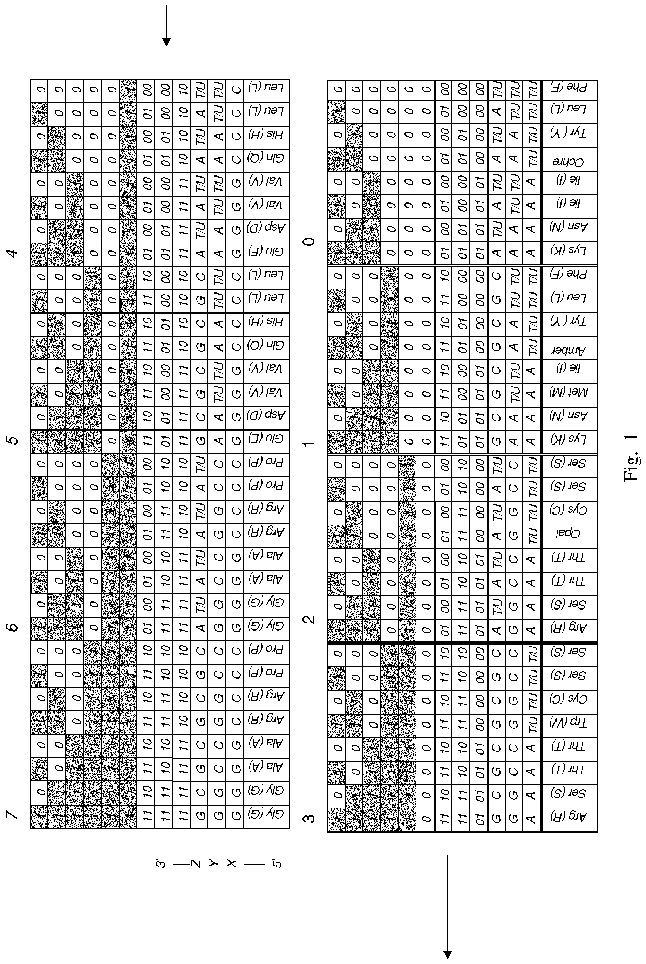

The positioning of the nitrogenous bases in the elementary basis graphically demonstrates that according to the first feature the horizontal pairs of the bases G=C (three hydrogen bonds, the strong position corresponding to the figure 1) and A=T/U (two hydrogen bonds, the weak position corresponding to the figure 0) are equivalent, and according to the second feature the vertical pairs G=A (two rings in the molecule, the strong position corresponding to the figure 1) and C=T/U (one ring in the molecule, the weak position corresponding to the figure 0) are equivalent.

The molecular genetic system for coding was generated by means of getting the tensor (Kronecker) product of the base by itself. For instance, a next-generation matrix $F^2$ for DNA of 4x4 will look as follows:

$$F^2 = \begin{bmatrix} GG & GC & CG & CC \\ GA & GT & CA & CT \\ AG & AC & TG & TC \\ AA & AT & TA & TT \end{bmatrix}$$

And a matrix of the $F^3$ generation for DNA of 8x8 will look as follows:

$$F^3 = \begin{bmatrix} GGG & GGC & GCG & GCC & CGG & CGC & CCG & CCC \\ GGA & GGT & GCA & GCT & CGA & CGT & CCA & CCT \\ GAG & GAC & GTG & GTC & CAG & CAC & CTG & CTC \\ GAA & GAT & GTA & GTT & CAA & CAT & CTA & CTT \\ AGG & AGC & ACG & ACC & TGG & TGC & TCG & TCC \\ AGA & AGT & ACA & ACT & TGA & TGT & TCA & TCT \\ AAG & AAC & ATG & ATC & TAG & TAC & TTG & TTC \\ AAA & AAT & ATA & ATT & TAA & TAT & TTA & TTT \end{bmatrix}$$

The number of elements in a matrix is substantiated by the number of combinations of triplets made of the four nitrogenous bases. Each of the triplets has its own specific character because it acts an eigenvalue of the matrix and has its own corresponding vector of this matrix. For instance, each of the three triplets AAC, ACA and CAA is coupled with its own vector, and, therefore, these triplets are considerably different in this respect.

Therefore, the molecular genetic system was formed as a result of an infinite number of repetitions of $F^n = F \otimes F^{n-1}$, where each **quadrant** of the matrix $F^{(n)}$ completely reproduces the matrix $F^{(n-1)}$ of the previous generation or the previous power. Each new-generation matrix conceals in itself the information about all of the previous generations (about the matrices in all of the previous powers). And the biggest multiplet matrix $F^{(\infty)}$ contains the information about all of the matrices with shorter multiplets. The higher the power of the matrices gets, the bigger is the number of combinations of nitrogenous bases that define the set of the unique elements of the structured system, and this set can be infinite. Thus the suggested system of elements can be used to receive, transmit, store and reproduce a vast volume of information.

In order to have an opportunity to process information using technical means, this same system can be formed in the binary system based on the index values of their sets in the columns and lines based on the combinations of the purine and pyramidine bases and the number of hydrogen bonds.

For instance, each triplet of the triplet matrix for DNA will be featured in the binary system as a hexagram, each of which will contain, for example, 1 byte of information, i.e., 6 bits in each byte.

$F^3_{\mathrm{DNA}} =$

| | | | | | | | |
|---|---|---|---|---|---|---|---|
| G 11<br>G 11<br>G 11 | C 10<br>G 11<br>G 11 | G 11<br>C 10<br>G 11 | C 10<br>C 10<br>G 11 | G 11<br>G 11<br>C 10 | C 10<br>G 11<br>C 10 | G 11<br>C 10<br>C 10 | C 10<br>C 10<br>C 10 |
| A 01<br>G 11<br>G 11 | T 00<br>G 11<br>G 11 | A 01<br>C 10<br>G 11 | T 00<br>C 10<br>G 11 | A 01<br>G 11<br>C 10 | T 00<br>G 11<br>C 10 | A 01<br>C 10<br>C 10 | T 00<br>C 10<br>C 10 |
| G 11<br>A 01<br>G 11 | C 10<br>A 01<br>G 11 | G 11<br>T 00<br>G 11 | C 10<br>T 00<br>G 11 | G 11<br>A 01<br>C 10 | C 10<br>A 01<br>C 10 | G 11<br>T 00<br>C 10 | C 10<br>T 00<br>C 10 |
| A 01<br>A 01<br>G 11 | T 00<br>A 01<br>G 11 | A 01<br>T 00<br>G 11 | T 00<br>T 00<br>G 11 | A 01<br>A 01<br>C 10 | T 00<br>A 01<br>C 10 | A 01<br>T 00<br>C 10 | T 00<br>T 00<br>C 10 |
| G 11<br>G 11<br>A 01 | C 10<br>G 11<br>A 01 | G 11<br>C 10<br>A 01 | C 10<br>C 10<br>A 01 | G 11<br>G 11<br>T 00 | C 10<br>G 11<br>T 00 | G 11<br>C 10<br>T 00 | C 10<br>C 10<br>T 00 |
| A 01<br>G 11<br>A 01 | T 00<br>G 11<br>A 01 | A 01<br>C 10<br>A 01 | T 00<br>C 10<br>A 01 | A 01<br>G 11<br>T 00 | T 00<br>G 11<br>T 00 | A 01<br>C 10<br>T 00 | T 00<br>C 10<br>T 00 |
| G 11<br>A 01<br>A 01 | C 10<br>A 01<br>A 01 | G 11<br>T 00<br>A 01 | C 10<br>T 00<br>A 01 | G 11<br>A 01<br>T 00 | C 10<br>A 01<br>T 00 | G 11<br>T 00<br>T 00 | C 10<br>T 00<br>T 00 |
| A 01<br>A 01<br>A 01 | T 00<br>A 01<br>A 01 | A 01<br>T 00<br>A 01 | T 00<br>T 00<br>A 01 | A 01<br>A 01<br>T 00 | T 00<br>A 01<br>T 00 | A 01<br>T 00<br>T 00 | T 00<br>T 00<br>T 00 |

Whereas, each triplet is built in the binary system starting from the lowest nitrogenous base since the lowest base is the element of the initial matrix $F^1$ of 2x2 by means of combining the binary code first according to the feature "number of hydrogen bonds" and then according to the feature "purine- pyramidine." A 5'-end is attached to the lowest base, and a 3'-end is attached to the upper base. It was also noticed that the motion of the electrons, as well as the readout, goes from bottom to top, i.e., from 5' -P04 (-) to the end 3' -OH(+).

Wherein, the described system of elements forms a "dominant/ recessive" trait which is important for the transfer of information. On the DNA and RNA level the dominant (stable) base is such a nitrogenous base that is present in both DNA and RNA, i.e., G, C, A. The recessive (changeable) base is a nitrogenous base that is contained either only in DNA (T) or only in RNA (U). Among the four nitrogenous bases, thymine (T) is set in opposition to the three other elements by nature because in the process of transition from DNA to RNA thymine (T) gets replaced by another nitrogenous base - uracil (U) - and is recessive. Therefore, considering the above feature, the presented molecular genetic system is similar to the Pennett square (1906) for polyhybrid crossing of organisms which functions in accordance with the Mendel's laws of polyhybrid crossing, which confirms the presence of natural multi-channel noise-immune coding of hereditary information in each organism. These squares are a graphic method of defining the genotype based on the combination of male and female gametes that was suggested by English biologist R. Punnett. However, Pennett's square feature not the eigenvalues of the matrices and their combinations but similar combinations of dominant and recessive gene alleles from the parents' reproductive cells/ gametes. In this situation, in cases where the information gets transferred the dominant traits will be coded with more reliable (stable) nitrogenous bases.

$$F^3 = \begin{bmatrix} GGG_1 & GGC_1 & GCG_1 & GCC_1 & CGG_1 & CGC_1 & CCG_1 & CCC_1 \\ GGA_1 & GGT_2 & GCA_1 & GCT_2 & CGA_1 & CGT_2 & CCA_1 & CCT_2 \\ GAG_1 & GAC_1 & GTG_3 & GTC_3 & CAG_1 & CAC_1 & CTG_3 & CTC_3 \\ GAA_1 & GAT_2 & GTA_3 & GTT_4 & CAA & CAT_2 & CTA_3 & CTT_4 \\ AGG_1 & AGC_1 & ACG_1 & ACC_1 & TGG_5 & TGC_5 & TCG_5 & TCC_5 \\ AGA_1 & AGT_2 & ACA_1 & ACT_2 & TGA_5 & TGT_6 & TCA_5 & TCT_6 \\ AAG_1 & AAC_1 & ATG_3 & ATC_3 & TAG_5 & TAC_5 & TTG_7 & TTC_7 \\ AAA_1 & AAT_2 & ATA_3 & ATT_4 & TAA_5 & TAT_6 & TTA_7 & TTT_8 \end{bmatrix}$$

Each triplet is marked by a certain color depending on the degree of nitrogenous base stability: red (lowest index: 1) features those triplets that have only stable bases that will not change both for DNA and RNA; orange (lowest index: 2) features those triplets in which only the upper (daughter) base will change and the two lower bases will remain stable; yellow (lowest index: 3) features those triplets in which the middle base will change, and the upper and lower bases will remain stable; green (lowest index: 4) features those triplets in which only the lower base will remain stable, and the upper and middle bases will change; light blue (lowest index: 5) features those triplets in which the lower (mother) base will change, and the upper and middle bases will remain stable; dark blue (lowest index: 6) features those triplets in which the lower and upper bases will change, and the middle base will remain stable; purple (lowest index: 7) features those triplets in which the lower and middle bases will change, and the upper base will remain stable; magenta (lowest index: 8) features those triplets in which all the bases will change.

The formed Pennett square visually reproduces the Sierpinsky carpet and illustrates the fractal structure of the elements in the molecular genetic system that repeats a part of itself. In other words, the present system has the same structure as its parts. Whereas, the Sierpinsky matrix reproduces the Hadamard matrix that defines the traits of noise-immune coding. Therefore, the elementary characteristics at all levels of life define the uniqueness of each element of the molecular genetic system, whose formation principle is based on, for instance, Walsh function that is widely used in noise-immune coding of information.

The application of fractals - i.e., matrices, each part of which represents the whole - allows us to form a molecular genetic system at various levels of life, using as elements of the matrix not only nitrogenous bases but also atoms, subatoms, amino acids and proteins that in their turn form (tissues, organs and construction material).

Information is transferred from DNA to RNA via a transport sequence of nitrogenous bases constructed based on the principle of their complementarity toward each other.

$$F_{DNA} = \begin{bmatrix} G & C \\ A & T \end{bmatrix} \rightarrow \begin{bmatrix} C & G \\ T & A \end{bmatrix} \rightarrow \begin{bmatrix} G & C \\ A & U \end{bmatrix} = F_{RNA}$$

When the operation $F^n = F \otimes F^{n-1}$ is repeated multiple times on the matrix of RNA nitrogenous bases, we will get a system of elements that is identical in structure but in which thymine (T) is replaced with uracil (U) that is also a pyramidine base with two hydrogen bonds:

$F_{\mathrm{RNA}}^{3}$ =

| | | | | | | | |
|---|---|---|---|---|---|---|---|
| G 11<br>G 11<br>G 11 | C 10<br>G 11<br>G 11 | G 11<br>C 10<br>G 11 | C 10<br>C 10<br>G 11 | G 11<br>G 11<br>C 10 | C 10<br>G 11<br>C 10 | G 11<br>C 10<br>C 10 | C 10<br>C 10<br>C 10 |
| A 01<br>G 11<br>G 11 | U 00<br>G 11<br>G 11 | A 01<br>C 10<br>G 11 | U 00<br>C 10<br>G 11 | A 01<br>G 11<br>C 10 | U 00<br>G 11<br>C 10 | A 01<br>C 10<br>C 10 | U 00<br>C 10<br>C 10 |
| G 11<br>A 01<br>G 11 | C 10<br>A 01<br>G 11 | G 11<br>U 00<br>G 11 | C 10<br>U 00<br>G 11 | G 11<br>A 01<br>C 10 | C 10<br>A 01<br>C 10 | G 11<br>U 00<br>C 10 | C 10<br>U 00<br>C 10 |
| A 01<br>A 01<br>G 11 | U 00<br>A 01<br>G 11 | A 01<br>U 00<br>G 11 | U 00<br>U 00<br>G 11 | A 01<br>A 01<br>C 10 | U 00<br>A 01<br>C 10 | A 01<br>U 00<br>C 10 | U 00<br>U 00<br>C 10 |
| G 11<br>G 11<br>A 01 | C 10<br>G 11<br>A 01 | G 11<br>C 10<br>A 01 | C 10<br>C 10<br>A 01 | G 11<br>G 11<br>U 00 | C 10<br>G 11<br>U 00 | G 11<br>C 10<br>U 00 | C 10<br>C 10<br>U 00 |
| A 01<br>G 11<br>A 01 | U 00<br>G 11<br>A 01 | A 01<br>C 10<br>A 01 | U 00<br>C 10<br>A 01 | A 01<br>G 11<br>U 00 | U 00<br>G 11<br>U 00 | A 01<br>C 10<br>U 00 | U 00<br>C 10<br>U 00 |
| G 11<br>A 01<br>A 01 | C 10<br>A 01<br>A 01 | G 11<br>U 00<br>A 01 | C 10<br>U 00<br>A 01 | G 11<br>A 01<br>U 00 | C 10<br>A 01<br>U 00 | G 11<br>U 00<br>U 00 | C 10<br>U 00<br>U 00 |
| A 01<br>A 01<br>A 01 | U 00<br>A 01<br>A 01 | A 01<br>U 00<br>A 01 | U 00<br>U 00<br>A 01 | A 01<br>A 01<br>U 00 | U 00<br>A 01<br>U 00 | A 01<br>U 00<br>U 00 | U 00<br>U 00<br>U 00 |

Therefore, the binary system of DNA triplets represented in form of hexagrams will be identical to the system of RNA triplets.

Similarly, information from RNA can be transferred via a transport sequence of nitrogenous bases of $_{T}$RNA to amino acids that build proteins. The triplet matrix $F_{\mathrm{RNA}}^{3}$ for RNA (as well all of the subsequent matrices divisible by 3) allows us to create a structural system for amino acids $F_{\mathrm{AA}}^{3}$, by assigning each triplet (codon) a certain amino acid according to the existing classification table of amino acids (https://en.wikipedia.org/wiki/Genetic_code). There are 64 codons and 20 corresponding amino acids: Ala - alanine, Arg - arginin, Asn - aspargine, Asp - aspartic acid, Cys - cystein, Gln - glutamine, Glu - glutamic acid, Gly - glycine, His - histidine, Ile - isoleucine, Leu - leucine, Lys - lysine, Met - methionine, Phe - phenylanine, Pro - proline, Ser - serine, Thr - threonine, Trp - tryptophan, Tyr - tyrosine, Val - valine.

$F_{\mathrm{AA}}^{3}$ =

| | | | | | | | |
|---|---|---|---|---|---|---|---|
| Gly (G) | Gly (G) | Ala (A) | Ala (A) | Arg (R) | Arg (R) | Pro (P) | Pro (P) |
| Gly (G) | Gly (G) | Ala (A) | Ala (A) | Arg (R) | Arg (R) | Pro (P) | Pro (P) |
| Glu (E) | Asp (D) | Val (V) | Val (V) | Gln (Q) | His (H) | Leu (L) | Leu (L) |
| Glu (E) | Asp (D) | Val (V) | Val (V) | Gln (Q) | His (H) | Leu (L) | Leu (L) |
| Arg (R) | Ser (S) | Thr (T) | Thr (T) | Trp (W) | Cys (C) | Ser (S) | Ser (S) |
| Arg (R) | Ser (S) | Thr (T) | Thr (T) | Opal | Cys (C) | Ser (S) | Ser (S) |
| Lys (K) | Asn (N) | Met (M) | Ile (I) | Amber | Tyr ( Y) | Leu (L) | Phe (F) |
| Lys (K) | Asn (N) | Ile (I) | Ile (I) | Ochre | Tyr ( Y) | Leu (L) | Phe (F) |

It is possible to use not only the standard genetic code but also known variations of the latter containing the 21st (selenocysteine) and 22nd (pyrrolysine) amino acids. The sequence of codons in a gene defines the sequence of amino acids in the polypeptide chain of protein that is being coded by this gene.

Any matrix formed based on the described rules with various own multiplet values is transformed into a sequence (linear representation) by means of known methods of element coding. In contrast to the hierarchical data structure, in which each element stores indicators about its previous generation, such indicators are not used in the linear representation. Among the methods of coding, the most widespread and effective one is the method with the Lebesgue curve (Z-order curve) and the Hilbert curve. The advantage of the Hilbert curve is its continuity, which means that the neighboring elements are positioned in a consecutive order. The advantage of the Z-order curve is its simplicity and fast calculation, that is why it is applied more often. In order to code elements using a Z-order curve the Morton code is applied, which is calculated for a Z-order curve by means of shifting and mixing bits in binary of each vector.

Fig. 1 shows one of the possible examples of the embodiment of the inventions using the molecular genetic system, i.e., a triplet matrix for DNA/ RNA and amino acids with binary indexing information assigned to each element of the matrix; as it is a triplet system, the indexing information consists of 64 unique hexagrams, the location of each of which is based on the characteristics of the nitrogenous bases of DNA/ RNA. For the purposes of better perception of the structure of the coding system, the 64 elements of the matrix are divided into eight octets that are numbered starting from the less stable elements of the system. The matrix is in the linear representation, in which each of the eight octets is positioned in a consecutive order one by one from the eighth to the first octet.

In the system used for coding of information, one can observe numerical representations applied in the code combination of check bits that either coincide with the fully orthogonal system of Walsh functions used in noise-immune coding for the discrete signal processing or have a constant value used to check the correctness of the system structure. Depending on the application of the methods of coding/ decoding being described, various structural matrix representations, featuring the main property of the Hadamard matrices, i.e., $FF^T = n1$, where F is a matrix of n elements -1 and +1, can be used. Besides, the numerical representations described above and the property of the Hadamard matrices are implemented in various structural representation of the triplet system:

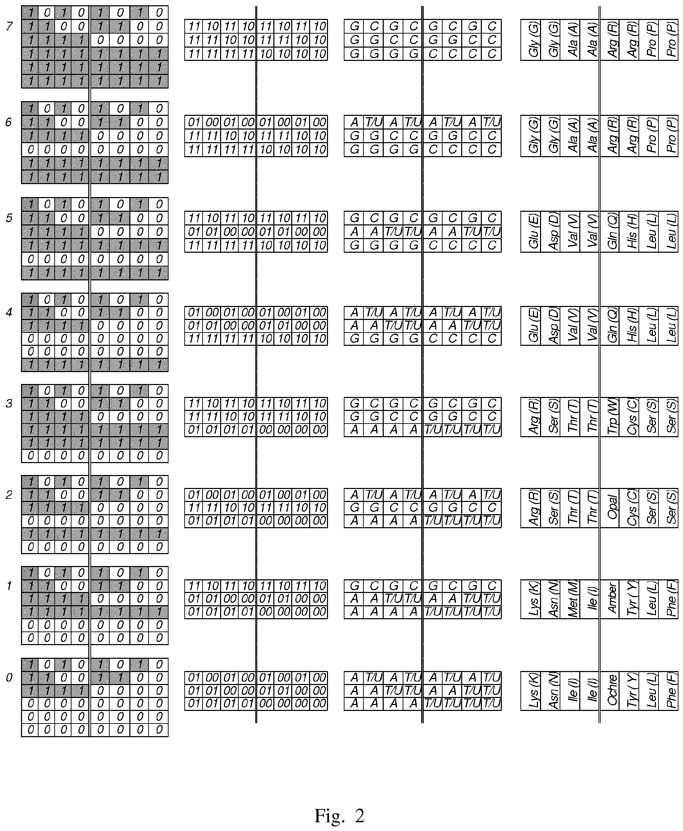

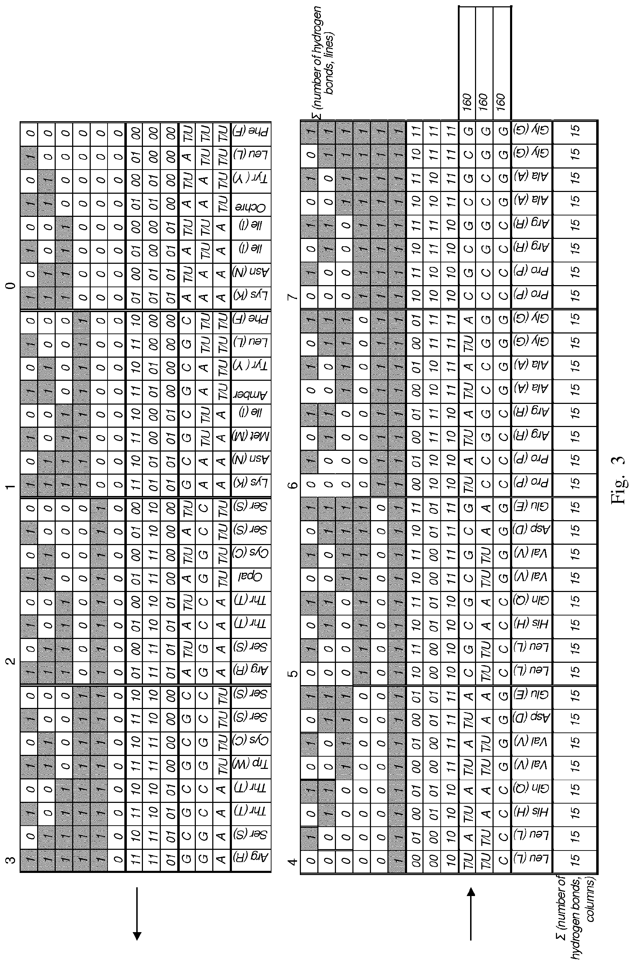

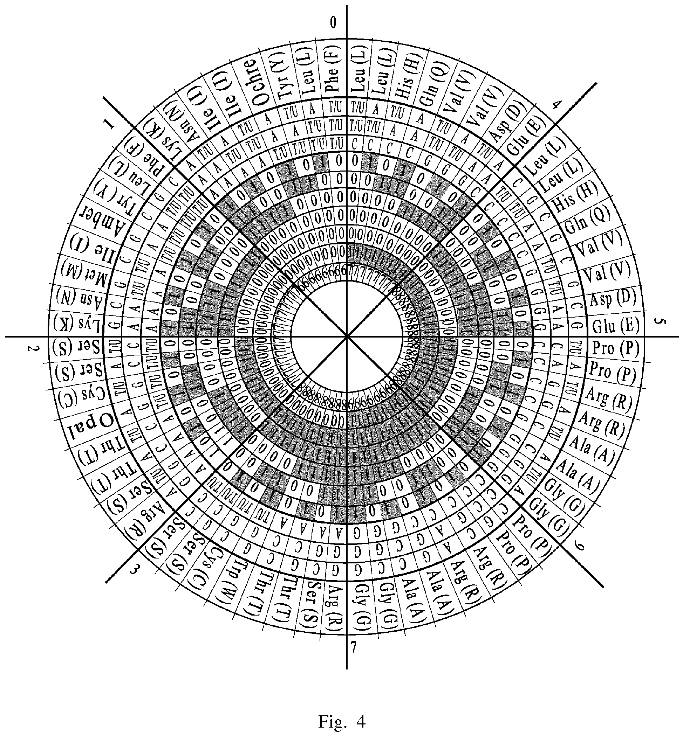

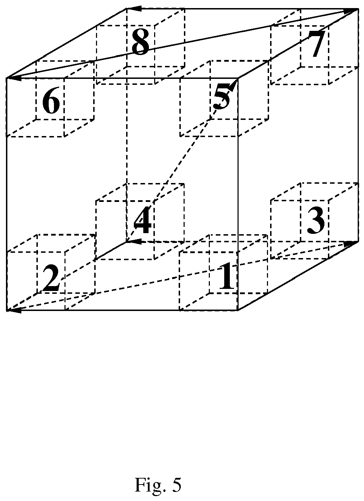

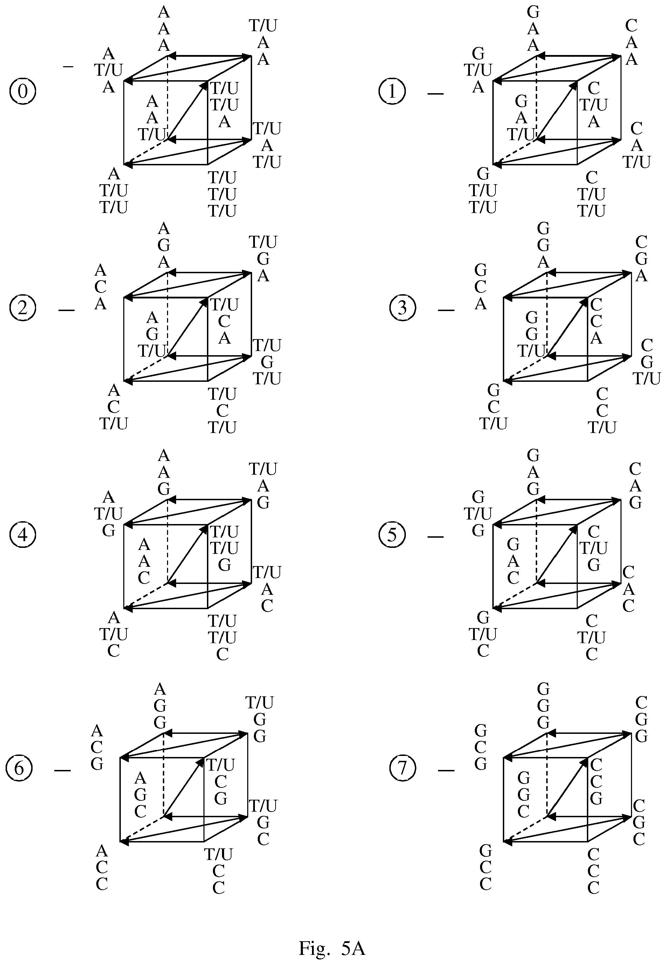

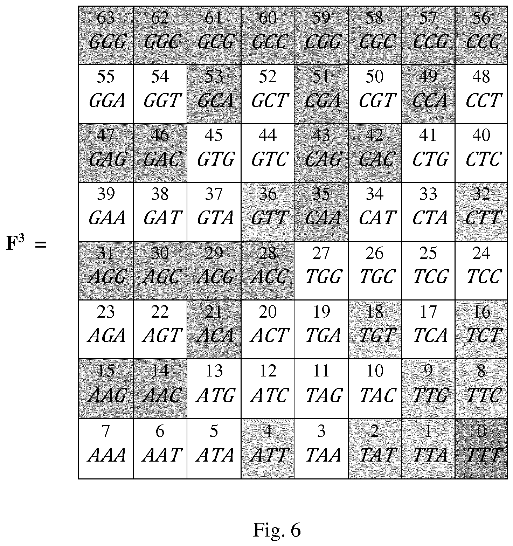

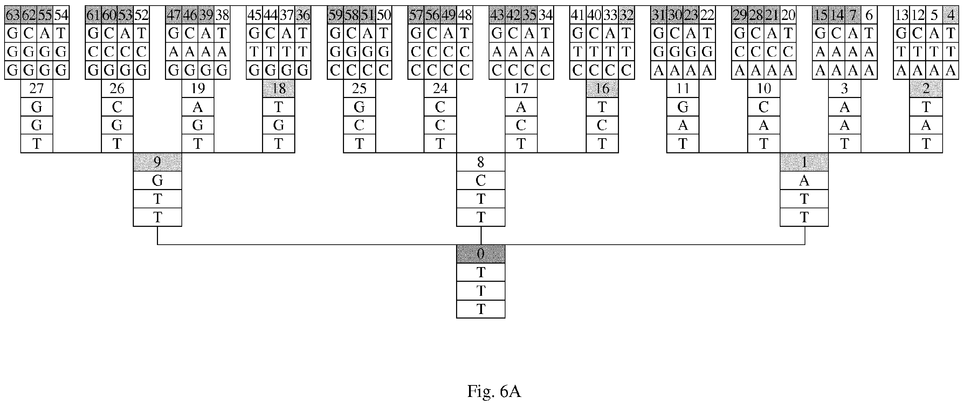

1) in the square matrix representation of the triplet matrix for DNA, RNA and amino acids (fig.2);
2) in the rectangular matrix representation of the triplet matrix for DNA, RNA and amino acids (fig. 3);
3) in the circle matrix representation of the triplet matrix for DNA, RNA and amino acids (fig. 4);
4) in the volume (cubical) matrix representation of the triplet matrix for DNA/ RNA (fig. 5, 5A);
5) in the tree structure matrix representation of the triplet matrix for DNA (fig. 6, 6A). Besides, in this example the numerical values of nitrogenous bases in the decimal system from 0 to 63 are formed by means of standard transformation from the binary system.

There can also be color and sound representations used for encoding images and sounds, respectively.

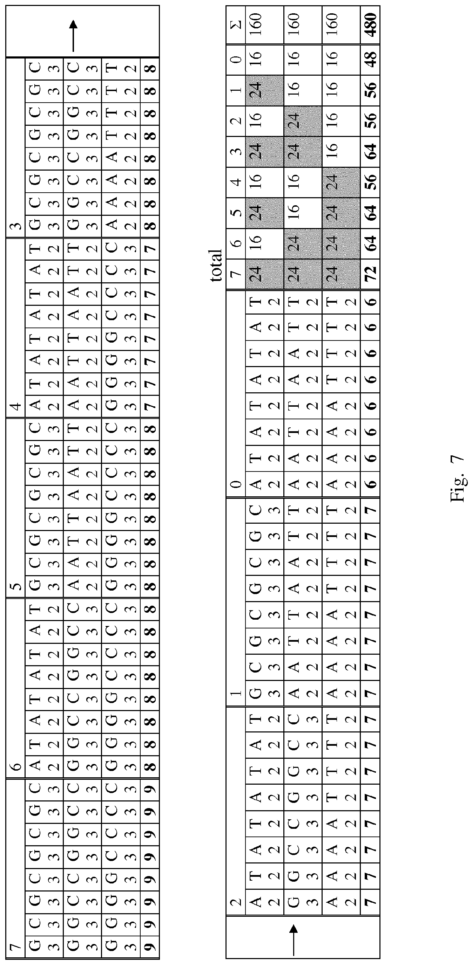

For instance, in the linear representation $F^3$ the number of hydrogen bonds for each of the 64 triplets changes from 9 to 6 with values decreasing toward the first octet of the matrix (fig. 7), besides, the number of hydrogen bonds in one octet is the same for all the triplets. The summarized values of the hydrogen bonds are either 24 or 16 in a horizontal line for each of the octets and coincide with the orthogonal system of Walsh functions. The total summarized values of all the hydrogen bonds in horizontal lines are the same and equal 160.

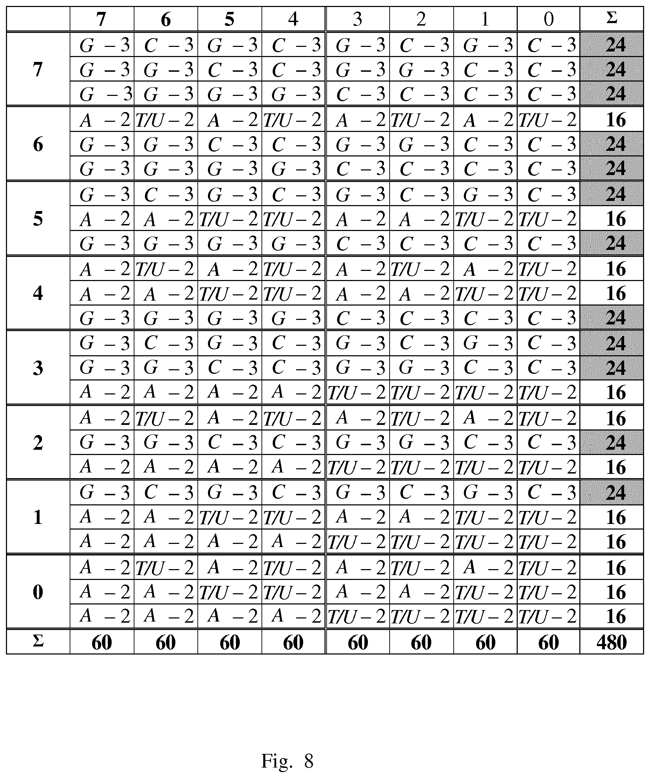

In the square representation $F^3$ the number of hydrogen bonds for each of the 64 triplets also changes from 9 to 6 with values decreasing toward the first octet of the matrix (fig. 8). Besides, the summarized values of the hydrogen bonds are either 24 or 16 in a horizontal line for each of the octets and coincide with the orthogonal system of Walsh functions. The total summarized values of all the hydrogen bonds in the columns are the same and equal 60. In the circular representation $F^3$ the number of hydrogen bonds for two triplets positioned in the opposite segments of the circle is the same when added up and equals 15 (fig. 4). For instance, nine hydrogen bonds of the triplets of the $8^{th}$ octet added to the six hydrogen bonds of the triplets of the opposite $1^{st}$ octet will be 15 in total.

Each nitrogenous base has a different number of atoms, among which are the different numbers of the atoms of carbon (C), hydrogen (H), nitrogen (N) and oxygen (O):

| Nitrogenous bases | Symbol of nitrogenous base | Number of atoms | | | | Total number of atoms |
|---|---|---|---|---|---|---|
| | | C | H | N | O | |
| Adenine | **A** | 5 | 5 | 5 | 0 | 15 |
| Cytosine | **C** | 4 | 5 | 3 | 1 | 13 |
| Guanine | **G** | 5 | 5 | 5 | 1 | 16 |
| Thymine | **T** | 5 | 6 | 2 | 2 | 15 |
| Uracil | **U** | 4 | 4 | 2 | 2 | 12 |

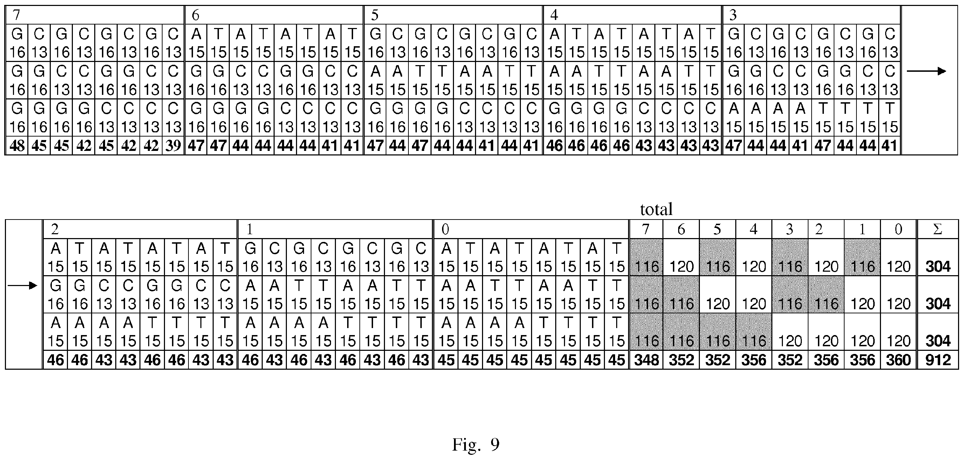

The triplet system $F^3$ based on the number the atoms has a pattern featured in the fig. 9. After adding up all the values of all the atoms of carbon (C), hydrogen (H), nitrogen (N) and oxygen (O) of each of the octets the value will be either 116 or 120 and coincide with the orthogonal system of Walsh functions. The total number of the atoms of carbon (C), hydrogen (H), nitrogen (N) and oxygen (O) in each horizontal line is the same and equals 944.

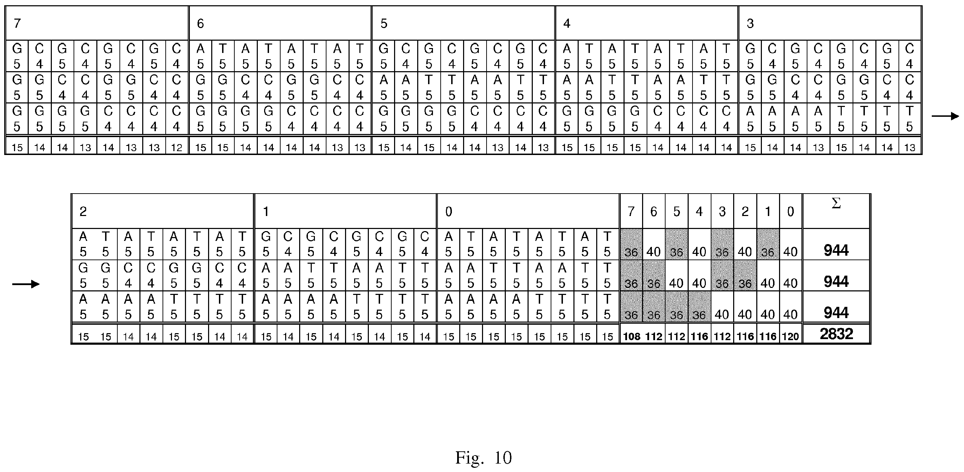

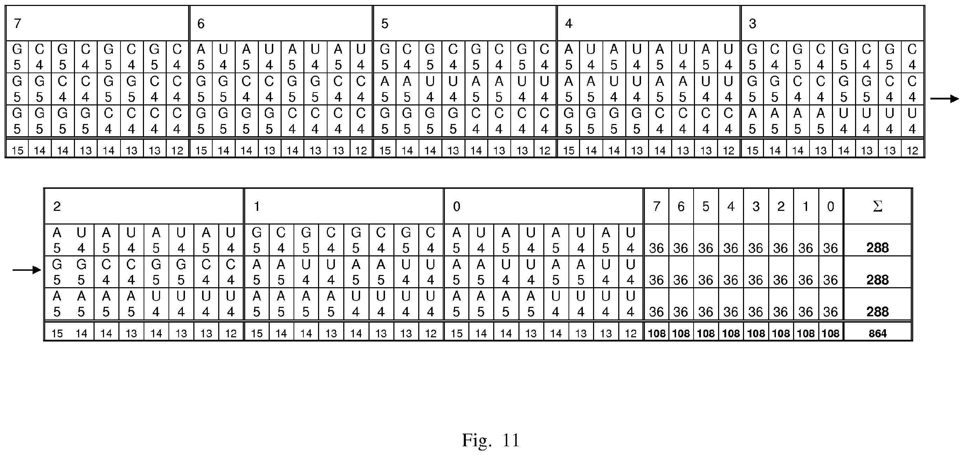

When considering the total number of the atoms of carbon (C) for DNA (fig. 10) in the linear representation of the triplet matrix, the total number of the atoms in one horizontal line is either 36 or 40 and coincides with the orthogonal system of Walsh functions. Whereas, when one considers the total number of the atoms of carbon (C) for RNA (fig. 11) in the linear representation of the triplet matrix, the total number of the atoms in each horizontal line is the same and equals 36.

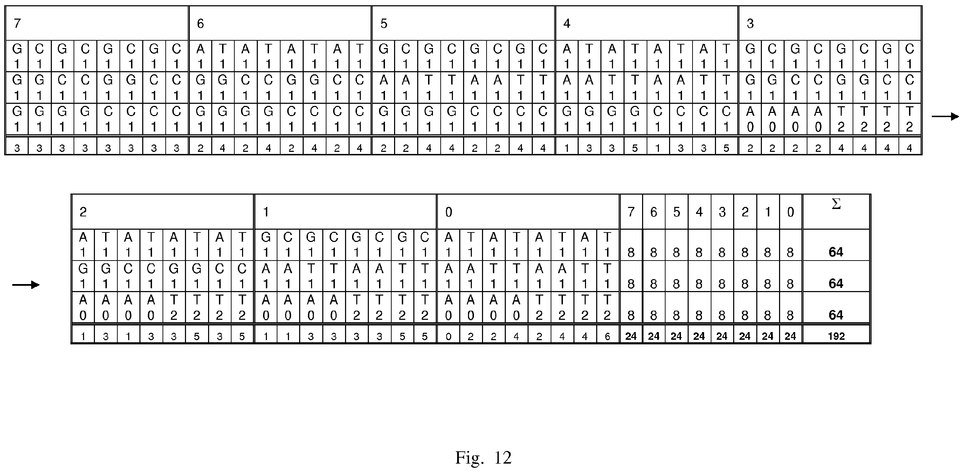

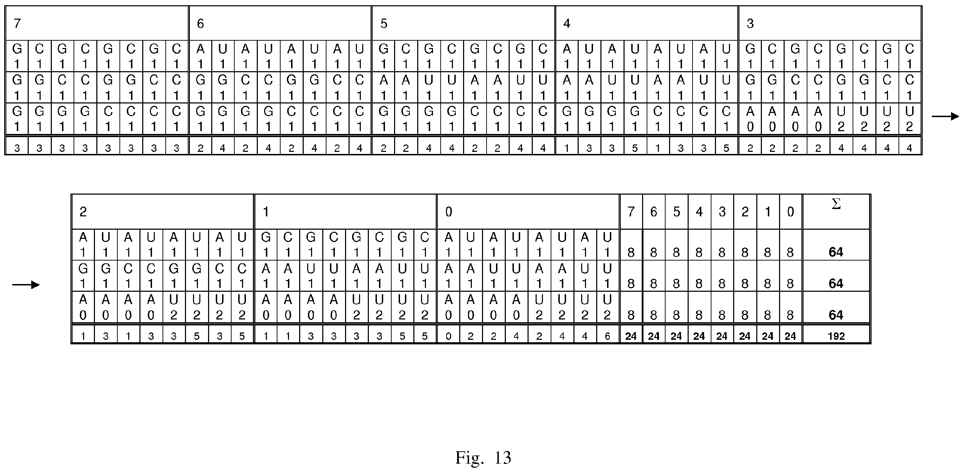

When considering the total number of the atoms of oxygen (O) for NA (fig. 12) and RNA (fig. 13) in the linear representation of the triplet matrix, the total numbers of the atoms in each horizontal line are the same both for DNA and RNA and equal 8.

Similarly, one can follow a pattern in the constructed system in its various representations based on the following characteristics:

1. The values of the electron and proton density in the atoms of the nitrogenous bases in general and in the atoms of carbon (C), hydrogen (H), nitrogen (N) and oxygen (O) of the nitrogenous bases in particular;
2. The values of the neutron density in the atoms of the nitrogenous bases, including the atoms of carbon (C), hydrogen (H), nitrogen (N) and oxygen (O) of the nitrogenous bases;
3. The difference between the values of the proton and neutron densities in the atoms of the nitrogenous bases, including the atoms of carbon (C), hydrogen (H), nitrogen (N) and oxygen (O) of the nitrogenous bases;
4. The "charge" of the nitrogenous bases ("+1" is "aMino" and "-1" is "Keto"). Each element of the system has its own electric charge: aMino (the positive charge) and Keto (the negative charge). Adenine and cytosine have the positive aMino charge, and guanine, thymine and uracil have the negative Keto charge;
5. The molecular density in the atoms of the nitrogenous bases, including the atoms of carbon (C), hydrogen (H), nitrogen (N) and oxygen (O) of the nitrogenous bases;
6. The density of the quarks in the protons of the atoms of the nitrogenous bases, including the atoms of carbon (C), hydrogen (H), nitrogen (N) and oxygen (O) of the nitrogenous bases;
7. The density of the quarks in the neutrons of the atoms of the nitrogenous bases, including the atoms of carbon (C), hydrogen (H), nitrogen (N) and oxygen (O) of the nitrogenous bases;
8. The density of the quarks in the protons and neutrons of the atoms of the nitrogenous bases, including the protons and neutron of the atoms of carbon (C), hydrogen (H), nitrogen (N) and oxygen (O) of the nitrogenous bases;
9. The density of the electronic valence band in the atoms of the nitrogenous bases.

Thus, by using at least one of the above listed characteristics additionally, one can also obtain numerical representations that will coincide with the Walsh function and could be used to check code combinations. As a result of the combination of nitrogenous bases and due to their characteristics, one will obtain a structural system of unique (non-recurrent) elements that characterizes the whole system. The logical structure is attested with the specific features explained above.

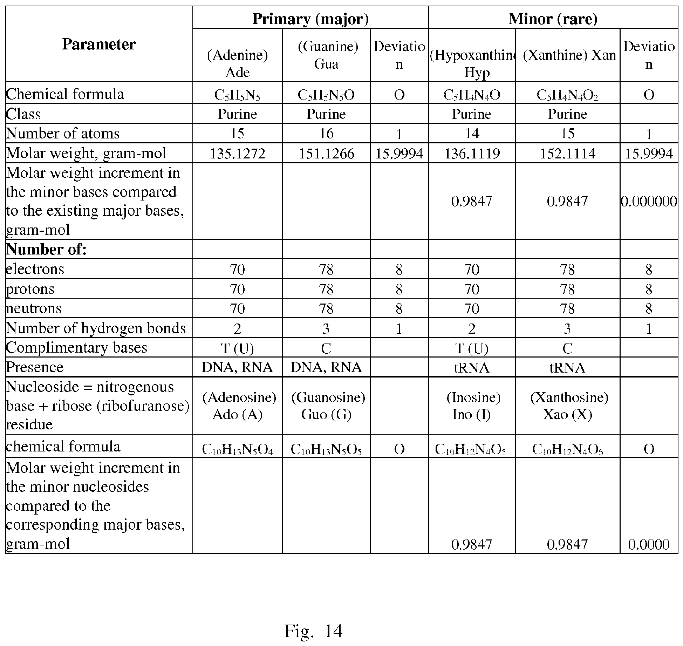

This example features major (commonly encountered) nitrogenous bases, however, one can also use minor (rarely encountered) nitrogenous bases in practical application. Each minor base that corresponds to a certain major base has similar characteristics used for the construction of a molecular genetic system. Therefore, the structure of a molecular genetic system will not be disrupted, in cases where a minor base is used. An example of the comparison between major and minor bases is featured in fig. 14. In cases where major and minor bases are used simultaneously, the information capacity of a molecular genetic system will increase.

Currently, the position of nucleotides in the codons at 5'-3' of the DNA strand is commonly indicated with X, Y and Z, where X is the prefix, Y is the root and Z is the ending. Therefore, a triplet on this strand will look as follows:

5'-X-Y-Z-3'.

A triplet on the strand 3'-5' complementary to the above example will, in its turn, look as follows:

3'-Z-Y-X-5'.

The triplet can be presented as the sum of the three vectors:

$$\vec{K} = \vec{X} + \vec{Y} + \vec{Z},$$

where

$\vec{K}$ - is the vector of the physical and biochemical "triplet" (codon) system;
$\vec{X}$ - is the first element of the physical and biochemical "triplet" (codon) system (nitrogenous base) presented in the form of a "prefix" unit vector;
$\vec{Y}$ - is the second element of the physical and biochemical "triplet" (codon) system (nitrogenous base) presented in the form of a "root" unit vector;
$\vec{Z}$ - is the third element of the physical and biochemical "triplet" (codon) system (nitrogenous base) presented in the form of an "ending" unit vector;
m - is the ordinal number of a triplet (or its elements) on the 5'-3' strand;
n -is the total number of triplets on the 5'-3' strand.

Each unit element of the vector "triplet" can, in its turn, be split into its components and written in the complex form as follows:

$$\vec{X} = x + ix ,$$

where

$\vec{X}$ - is the first element of the physical and biochemical "triplet" system (nitrogenous base) presented in the form of a "prefix" unit vector;
x - is the real part of a complex number which corresponds to the axis Hb (axis of hydrogen bonds);
*i* - is the imaginary unit for the "prefix" element;
*ix* - is the imaginary part of a complex number which corresponds to the axis PP (class of the nitrogenous base).

The set of unit vectors can be presented in a unit circle on a complex plane. Similarly, the elements of a Y-type triplet ("root") and a Z-type triplet ("ending") can be presented.

Therefore, one is able to write down the triplet in the m position expressed as follows:

$$\vec{K} = \vec{X} + \vec{Y} + \vec{Z} = (x + ix) + (y + jy) + (z + kz) ,$$

where
*i, j, k* - are the imaginary units with the following properties:
in the matrix form

$$K_m = \begin{bmatrix} x_m & y_m & z_m \\ ix_m & jy_m & kz_m \end{bmatrix}$$

Since the number of allocations with repetition from n for k is calculated based on the formula $A_n^k = n^k$, the three nitrogenous bases combined in a triplet form 64 different codons which is equivalent to the logarithmic expression: $\log_4 64 = 3$.

Example 1. The implementation in the method of coding described above for recording check bits of the logical operation "modulo addition" (ab), in which the following rule is applied for binary modulo addition: the result equals "0", when both operands (a) and (b) are different, and in all other cases the result equals "1":

| a b | ab |
|---|---|
| 0 0 | 0 |
| 1 0 | 1 |
| 0 1 | 1 |

(continued)

| a b | ab |
|---|---|
| 1 1 | 0 |

For the purposes of the ternary modulo 2 addition (X,Y,Z) the following rule is used: the result equals "0", when there are no operands that equal "1" or when their number is even, in all other cases the result equals "0":

| **X Y Z** | **⊕(X,Y,Z)** |
|---|---|
| 0 0 0 | 0 |
| 1 0 0 | 1 |
| 0 1 0 | 1 |
| 1 1 0 | 0 |
| 0 0 1 | 1 |
| 1 0 1 | 0 |
| 0 1 1 | 0 |
| 1 1 1 | 1 |

This method of recording is based on splitting major nitrogenous bases (A, C, G, T, U) or their minor equivalents into three bits:

- "p" bit of the nitrogenous base class (purine or pyrimidine);
- "h" bit of the number of hydrogen bonds of a nitrogenous base (2 or
- "e" bit of the nitrogenous base group (aMino or Keto).

Whereas, the two first data bits (p - purines or pyrimidines and h - the number of hydrogen bonds) represent variable values, and the third bit - the e check bit - represents the result of the logical operation.

The table below features the correlations of the bit values of nitrogenous bases for the implementation of the method of recording logical elements, where "p" is the class, i.e., purine or pyrimidine, "h" is the number of hydrogen bonds which can be 2 or 3, and "e" is the group, i.e., Keto or aMino.

Table

| # | Nitrogenous base | Class | Number of hydrogen bonds | Group | p (a) | h (b) | e (ab) |
|---|---|---|---|---|---|---|---|
| 1 | A | purine | 2 | aMino | 1 | 0 | 1 |
| 2 | C | pyrimidine | 3 | aMino | 0 | 1 | 1 |
| 3 | G | purine | 3 | Keto | 1 | 1 | 0 |
| 4 | T | pyrimidine | 2 | Keto | 0 | 0 | 0 |
| 5 | U | pyrimidine | 2 | Keto | 0 | 0 | 0 |

As one can see from the table, one can record 3 (two data bits and one check bit) bits of information using one nitrogenous base, moreover, when this method is used to record logical elements as triplets (codons), the binary and ternary modulo 2 additions proceed simultaneously.

| Nitrogenous base | p (a) | h (b) | e (ab) |
|---|---|---|---|
| 3' | | | |
| \| | | | |
| Z | pz | hz | $\sum z$ |
| Y | py | hy | $\sum y$ |
| X | px | hx | $\sum x$ |
| XYZ | $\sum p$ | $\sum h$ | $\sum\sum$ |

(continued)

| Nitrogenous base | p (a) | h (b) | e (ab) |
|---|---|---|---|
| \| 5’ | | | |

Therefore, 9 bits of information (6 data bits of variable values and 3 check (result) bits) can be recorded with one nitrogenous base in the above example.

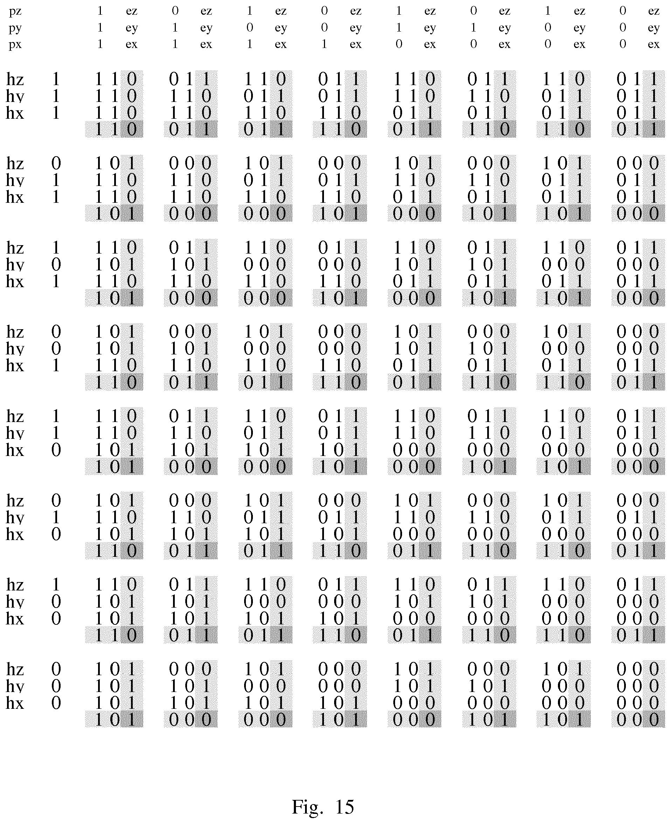

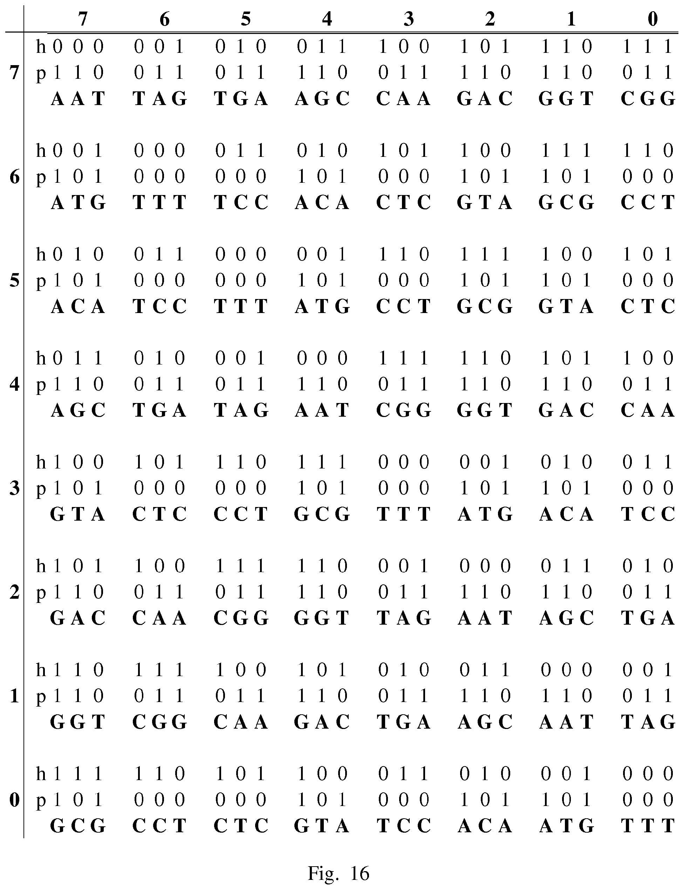

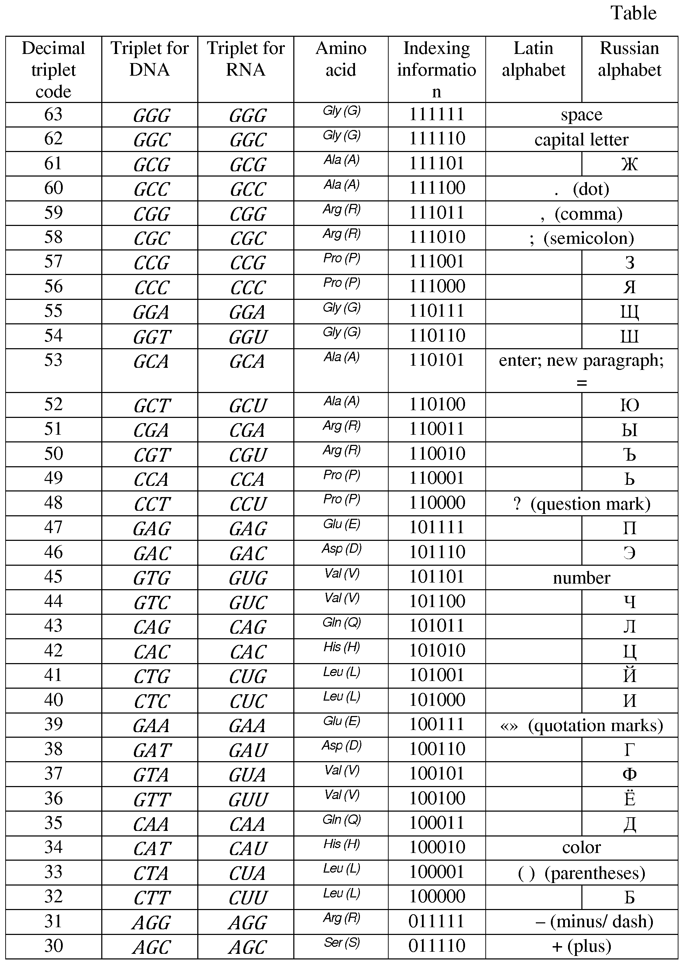

When comparing the total number of the values of the Boolean function (fig. 15) for the resultant triplet vector e (∑x∑y∑z), which is 8 (from 000 to 111) with the total number of values for the resultant triplet vector XYZ (∑p∑h∑∑), which is 4 (000; 011; 101; 110), we obtain the total number of bit-to-bit values in the triplet expression of nitrogenous bases, which is 16 (fig. 16).

By the virtue of the fact that 16 combinations are obtained in total for 64 triplets, there is an opportunity to pack the expression of 16 sets "tighter" using duplets instead of triplets (see tables 2a and 2b).

Table 2a.
Forming 16 duplets (by two nitrogenous based)

| | **G** | **A** | **C** | **T** |
|---|---|---|---|---|
| **G** | GG | GA | GC | GT |
| **A** | AG | AA | AC | AT |
| **C** | CG | CA | CC | CT |
| **T** | TG | TA | TC | TT |

Table 2b.
Forming 16 duplets (the duplets are numbered in accordance with their position in the table 2a)

| | **G** | **A** | **C** | **T** |
|---|---|---|---|---|
| **G** | 15 | 14 | 13 | 12 |
| **A** | 11 | 10 | 9 | 8 |
| **C** | 7 | 6 | 5 | 4 |
| **T** | 3 | 2 | 1 | 0 |

As a result of this operation, we make one spot available for a nitrogenous base in the triplet (Table 3) and can use it as a control value of one of the four states: G (11); C (01); A (10) and T (00).

For instance,
G (11) - is the symbol given in bold
C (01) - is the symbol given in italics (oblique font)
A (10) - is the underlined symbol
T (00) - is the standard symbol

Table 3.
Correlation between the values of the resultant vectors (16 sets of codons) and the triplet and duplet expressions

| **№** | | **Triplet** | | **Numerical code** | **Duplet** |
|---|---|---|---|---|---|
| 1 | T | T | T | 0 | TT |
| 2 | T | C | C | 1 | TC |
| 3 | T | A | G | 2 | TA |
| 4 | T | G | A | 3 | TG |
| 5 | C | T | C | 4 | CT |
| 6 | C | C | T | 5 | CC |
| 7 | C | A | A | 6 | CA |
| 8 | C | G | G | 7 | CG |

(continued)

| № | Triplet | | | Numerical code | Duplet |
|---|---|---|---|---|---|
| 9 | A | T | G | 8 | AT |
| 10 | A | C | A | 9 | AC |
| 11 | A | A | T | 10 | AA |
| 12 | A | G | C | 11 | AG |
| 13 | G | T | A | 12 | GT |
| 14 | G | C | G | 13 | GC |
| 15 | G | A | C | 14 | GA |
| 16 | G | G | T | 15 | GG |
| | - | - | > | | |
| 5' | X | Y | Z | 3' | |

Example 2 Coding the word "МИР" (*Russian:* "WORLD") (without checking for errors)

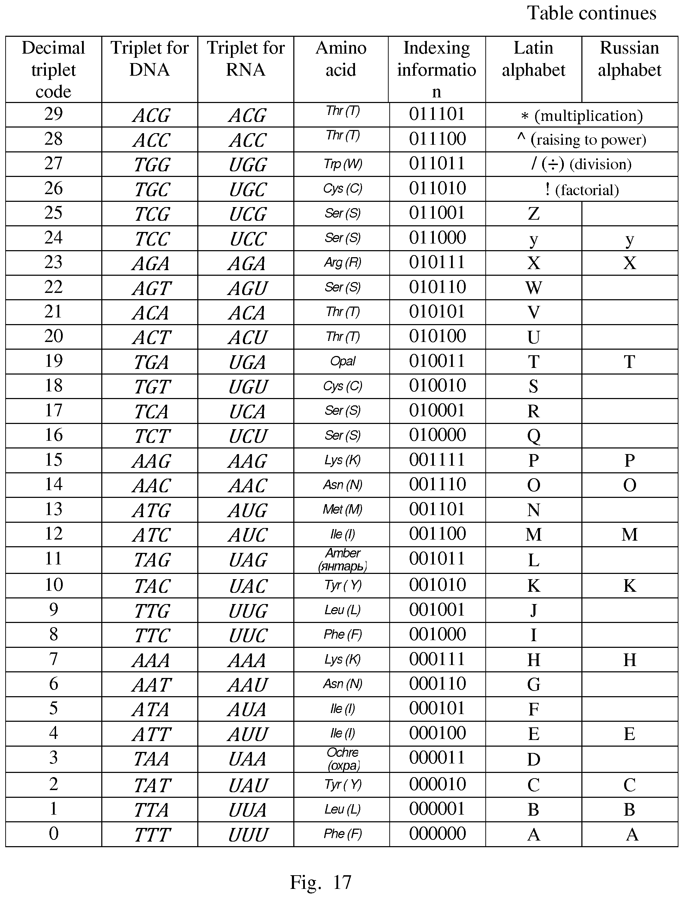

Fig. 17 shows the correlations between the triplet codes of DNA (RNA) and the symbols of the Latin and Russian alphabets. The record proceeds from the 5'-end to the 3'-end. In the featured example, three nitrogenous bases correspond to one logical element.

The code combination can be expressed in different variations:

1) linear:

5' - phe - phe - phe - 3'
5' - X - Z - X' - 3'

2) block (vertical):

| | |
|---|---|
| 3' | 3' |
| \| | \| |
| Z | phe |
| Y | phe |
| X | phe |
| \| | \| |
| 5' | 5' |

3) block (horizontal):

Z
5'- Y -3'
X
phe
5'-phe-3'
phe

The word "МИР" is split into logical elements "M", "И" and "P". Each element is associated with its unique symbol of the alphabet completed on the basis of the molecular genetic system of triplets (fig. 17) constructed in accordance with the principle described above. Each symbol is assigned an element of the system and supplemented by indexing information.

5' – A T C C T C A A G – 3'

5' – 1 0 1 0 0 0 0 1 1 – 3'
5' – A – T – C – 3'
**M**

5' – 0 1 1 0 0 0 0 1 1 – 3'
5' – C – T – C – 3'
**И**

5' – 1 0 1 1 0 1 1 1 0 – 3'
5' – A – A – G – 3'
**P**

Then a code combination is defined based on the values of the function of the modulo 2 addition:

| | | | | | |
|---|---|---|---|---|---|
| | | p | h | E | |
| | C | 0 | 1 | 1 | hz |
| **M** | T | 0 | 0 | 0 | hy |
| | A | 1 | 0 | 1 | hx |
| | | 1 | 1 | 0 | |
| | | px | py | pz | |

| | | | | | |
|---|---|---|---|---|---|
| | | p | h | e | |
| | C | 0 | 1 | 1 | hz |
| **И** | T | 0 | 0 | 0 | hy |
| | C | 0 | 1 | 1 | hx |
| | | 0 | 0 | 0 | |
| | | px | py | pz | |

| | | | | | |
|---|---|---|---|---|---|
| | | p | h | e | |
| | G | 1 | 1 | 0 | hz |
| **P** | A | 1 | 0 | 1 | hy |
| | A | 1 | 0 | 1 | hx |
| | | 1 | 1 | 0 | |
| | | px | py | pz | |

Due to the fact that when symbols are recorded using nitrogenous bases, this procedure is not subject to error checking, each nitrogenous base will actually correlate with 2 bits of information. Therefore, the word "МИР " will have 9 nitrogenous bases or 18 bits.

Example 3 Coding the word "МИР" with checking for errors. Just like in the previous example, this record also proceeds from the 5'-end to the 3'-end. Whereas, the given example features that one logical element correlates with six nitrogenous bases (X, Y, Z, X', Y', Z'): 3 nitrogenous bases (X, Y, Z) are intended for recording the information, and the other 3 nitrogenous bases (X', Y', Z') are intended for recording a control (check) code.

In order for us to be able to verify the way in which a record is read accurately, we shall refer to the data specified in fig. 16 that features the values of the resultant vectors and their triplet interpretation made of 16 sets of codons.

Therefore, coding proceeds based on the following algorithm:

1. The array of information to be coded is divided into logically complete chunks: the word specified in the example is divided into letters;
2. Each chunk is associated with a triplet DNA (RNA) code 5'-XYZ-3' according to fig. 17, and the code combinations of data bits (variable values that encode a particular characteristic of the nitrogenous base) are arranged in the form of a matrix.
3. Each code combination of data bits is supplemented by check bits that are the resulting values for binary modulo 2 addition of the data bit code combinations, for the ternary modulo 2 addition of the data bit code combinations, and the sum of the sums (the check of checks).
4. The triplet 5'-X'Y'Z'-3' is correlated to the check bits (fig. 16), and the sequence of the check bits of the binary and ternary addition and the final check (the sum of sums) is formed.
5. The final expression of the symbol is formed out of six nitrogenous bases; the expression consists of the primary (5'-XYZ-3') and supplementary triplets 5'-X'Y'Z'-3'):

| | | | | | | | | | | | | | |
|---|---|---|---|---|---|---|---|---|---|---|---|---|---|
| 5' | – | p H e | – | p h e | – | p h e | – | p h | – | p h | – | p h | – 3' |
| 5' | – | X | – | Y | – | Z | – | X' | – | Y' | – | Z' | – 3' |

character (symbol) result of check

In order to prove the noise-immunity of the suggested method that allows us to minimize the losses of information in case of errors, one shall use error detection methods. One of the most popular error detection methods is the even parity check that is done for lines and columns of the matrix formed based on the code combinations of the data bits - example 4. If the number of units is even, one shall add "0", if the number of units is odd, one shall add "1". The final step of the even parity check is the combined check of all the lines and columns.

If the result of the all checks is "0", the combination is correct. If there is an error, the result of the check will be "1". The incorrect bit will be at the intersection of the line and the column in which "1" was discovered as a result of the check.

The word "МИР" was coded with the possibility of an error check in the following sequence:

5' – **A** **T** **C** G A C **C** **T** **C** C T C **A** **A** **G** G G T – 3'

| | | | | | | | | | | | | | | | | | | | | | |
|---|---|---|---|---|---|---|---|---|---|---|---|---|---|---|---|---|---|---|---|---|---|
| M | 5' – | 1 | 0 | 1 | – | 0 | 0 | 0 | – | 0 | 1 | 1 | – | 1 | 1 | – | 1 | 0 | – | 0 | 1 – 3' |
| | 5' – | | A | | – | | T | | – | | C | | – | G | | – | A | | – | C | – 3' |

M check data

| | | | | | | | | | | | | | | | | | | | | | |
|---|---|---|---|---|---|---|---|---|---|---|---|---|---|---|---|---|---|---|---|---|---|
| И | 5' – | 0 | 1 | 1 | – | 0 | 0 | 0 | – | 0 | 1 | 1 | – | 0 | 1 | – | 0 | 0 | – | 0 | 1 – 3' |
| | 5' – | | C | | – | | T | | – | | C | | – | C | | – | T | | – | C | – 3' |

И check data

| | | | | | | | | | | | | | | | | | | | | | |
|---|---|---|---|---|---|---|---|---|---|---|---|---|---|---|---|---|---|---|---|---|---|
| P | 5' – | 1 | 0 | 1 | – | 1 | 0 | 1 | – | 1 | 1 | 0 | – | 1 | 1 | – | 1 | 1 | – | 0 | 0 – 3' |
| | 5' – | | A | | – | | A | | – | | G | | – | G | | – | G | | – | T | – 3' |

P check data

The matrix of data bits and check bits, as well as the matrix for an even parity check look as follows:

| | | | | | | | | | | | | | |
|---|---|---|---|---|---|---|---|---|---|---|---|---|---|
| | | p | h | E | | | | | | | | | |
| | C | 0 | 1 | **1** | hz | **1** | **0** | **1** | h | 0 | 1 | 1 | 0 |
| **M** | T | 0 | 0 | **0** | hy | **1** | **1** | **0** | p | 0 | 0 | 0 | 0 |
| | A | 1 | 0 | **1** | hx | G | A | C | | 1 | 0 | 1 | 0 |
| | | **1** | **1** | **0** | | | | | | 1 | 1 | 0 | |
| | | px | py | pz | | | | | | 0 | 0 | 0 | |

| | | | | | | | | | | | | | |
|---|---|---|---|---|---|---|---|---|---|---|---|---|---|
| | | p | h | e | | | | | | | | | |
| | C | 0 | 1 | **1** | hz | **1** | **0** | **1** | h | 0 | 1 | 1 | 0 |
| **И** | T | 0 | 0 | **0** | hy | **0** | **0** | **0** | p | 0 | 0 | 0 | 0 |
| | C | 0 | 1 | **1** | hx | C | T | C | | 0 | 1 | 1 | 0 |
| | | **0** | **0** | **0** | | | | | | 0 | 0 | 0 | |
| | | px | py | pz | | | | | | 0 | 0 | 0 | |

| | | | | | | | | | | | | | |
|---|---|---|---|---|---|---|---|---|---|---|---|---|---|
| | | p | h | e | | | | | | | | | |
| | G | 1 | 1 | **0** | hz | **1** | **1** | **0** | h | 1 | 1 | 0 | 0 |
| **P** | A | 1 | 0 | **1** | hy | **1** | **1** | **0** | p | 1 | 0 | 1 | 0 |
| | A | 1 | 0 | **1** | hx | G | G | T | | 1 | 0 | 1 | 0 |
| | | **1** | **1** | **0** | | | | | | 1 | 1 | 0 | |
| | | px | py | pz | | | | | | 0 | 0 | 0 | |

The example 4 features a method of recording symbols using nitrogenous bases (with 4 states of the symbol and a check for errors based on a matrix code). One logical element correlates with six nitrogenous bases (X, Y, Z, X', Y', Z') containing 15 bits of information, among which are 3 nitrogenous bases (X, Y, Z) intended for the recording of the information and containing 9 bits, 2 nitrogenous bases (X', Y') containing 4 bits of information and intended for the recording o the check code, and one nitrogenous base (Z') containing 2 bits and intended for the control code.

The density of the information being recorded here is 15 bits/ 6 nitrogenous bases and is therefore 2.5 bits per a nitrogenous base.

The code information and the check algorithm is formed according to the same method that has been shown in the example 3. The example is special since the check values are formed using not a triplet code, but a duplet code (fig. 16). As a result, one nitrogenous base (Z') becomes available for the control code that is used for supplementary information.

For instance, the control code that falls on the nitrogenous base (Z') may be presented in the form of the following coding, as in the example 1:

G (11) - is the symbol given in bold
C (01) - is the symbol given in italics (oblique font)
A (10) - is the underlined symbol
T (00) - is the standard symbol in the text

5' – p H e – p h e – p h e – p h – p h – p h – 3'

5' – X – Y – Z – X' – Y' – Z' – 3'

character result of check contr. code

Thus, the word "МИР " can be encoded with the possibility of error checking and the possibility of being written in a special font (we used italics in the example). Word: « *М И Р* »

5' – A T C G A C C T C C T C A A G G G C – 3'

| | | | | | | | | |
|---|---|---|---|---|---|---|---|---|
| M | 5' – | 1 0 1 | 0 0 0 | 0 1 1 | 1 1 | 1 0 | 0 1 | – 3' |
| | 5' – | A – | T – | C – | G – | A – | C | – 3' |

M check

| | | | | | | | | |
|---|---|---|---|---|---|---|---|---|
| И | 5' – | 0 1 1 | 0 0 0 | 0 1 1 | 0 1 | 0 0 | 0 1 | – 3' |
| | 5' – | C – | T – | C – | C – | T – | C | – 3' |

И check

| | | | | | | | | |
|---|---|---|---|---|---|---|---|---|
| P | 5' – | 1 0 1 | 1 0 1 | 1 1 0 | 1 1 | 1 1 | 0 1 | – 3' |
| | 5' – | A – | A – | G – | G – | G – | C | – 3' |

P check

| | | | | | | | | | | | | | | |
|---|---|---|---|---|---|---|---|---|---|---|---|---|---|---|
| | | p | h | e | | | | | | | | | | |
| M | C | 0 | 1 | 1 | hz | 1 | 0 | 1 | h | 0 | 1 | 1 | 0 |
| | T | 0 | 0 | 0 | hy | 1 | 1 | 0 | p | 0 | 0 | 0 | 0 |
| | A | 1 | 0 | 1 | hx | G | A | C | | 1 | 0 | 1 | 0 |
| | | 1 | 1 | 0 | | | | | | 1 | 1 | 0 | |
| | | px | py | pz | | | | | | 0 | 0 | 0 | |

| | | | | | | | | | | | | | |
|---|---|---|---|---|---|---|---|---|---|---|---|---|---|
| | | p | h | e | | | | | | | | | |
| И | C | 0 | 1 | 1 | hz | 1 | 0 | 1 | h | 0 | 1 | 1 | 0 |
| | T | 0 | 0 | 0 | hy | 0 | 0 | 0 | p | 0 | 0 | 0 | 0 |
| | C | 0 | 1 | 1 | hx | C | T | C | | 0 | 1 | 1 | 0 |
| | | 0 | 0 | 0 | | | | | | 0 | 0 | 0 | |
| | | px | py | pz | | | | | | 0 | 0 | 0 | |

| | | | | | | | | | | | | | |
|---|---|---|---|---|---|---|---|---|---|---|---|---|---|
| | | p | h | e | | | | | | | | | |
| P | G | 1 | 1 | 0 | hz | 1 | 1 | 0 | h | 1 | 1 | 0 | 0 |
| | A | 1 | 0 | 1 | hy | 1 | 1 | 0 | p | 1 | 0 | 1 | 0 |
| | A | 1 | 0 | 1 | hx | G | G | T | | 1 | 0 | 1 | 0 |
| | | 1 | 1 | 0 | | | | | | 1 | 1 | 0 | |
| | | px | py | pz | | | | | | 0 | 0 | 0 | |

The information being coded in accordance with the described method is recorded, among other things, by means of splitting it into short fragments of DNA, in which information about the beginning and the end of the chunk of data is recorded. Whereas, one nucleotide on one level of the hydrogen bonds and base codes at least two bits of data. When information is being recorded, blocks are synthesized from separate nucleotides by means of an inkjet DNA printer. In order for specific information to be recorded, the required fragments of DNA are synthesized; such fragments are preliminary multiplied and identified by means of a sequencing device, e.g. Illumina HiSeq. Thanks to the fact that each block has its own address, information can be stored in the form of a mix of short nucleotide sequences and not as a single strand of DNA. Such a method allows us to store an almost unlimited amount of data. In order to read synthesized fragments of DNA, one can use known sequencing technologies and special software intended for the translation of the genetic code back into the binary data based on the suggested molecular genetic system.

The molecular genetic system described above is also used for the decoding of information. For the purposes of decoding, machine-readable information is divided into equal parts consisting of an i+k+m number of zeros and ones that would be the indices of the logically complete chunks of the information to be decoded; these parts consist of i data bits, k check bits and m control bits. Then each part is associated with a multiplet consisting of the n number of nitrogenous bases and being an element of the molecular genetic system, thus defining the sequence of logically complete chunks of the information being decoded.

Therefore, when implementing the suggested method of coding and decoding information using the molecular genetic system in the form of square matrices, structured according to the characteristics of the nitrogenous bases of nucleotides, one can reach the claimed technical result, which includes handling a vase volume of information without it being distorted or changed and ensuring that a digital signal is coded and decoded in such a way that it remains immune to the noise when being transmitted via software tools.

## Claims

1. A method of coding information, whereby

   - the array of the information to be coded is divided into logically complete chunks each of which is associated with at least one element of the molecular genetic system used for the coding;
   - the set of the elements in the system is supplemented by relevant indexing information consisting of i data bits;
   - each code combination of data bits is supplemented by a combination of k check bits which is defined depending on i data bits combination;

   ***the method comprising***

   - recording of each i+k bit data in binary form as a multiplet consisting of the n number of nitrogenous bases / corresponding amino acids; the multiplet is an element of the molecular genetic system, consisting of a base representing a four-nitrogenous-base matrix $\begin{bmatrix} G & C \\ A & T/U \end{bmatrix}$; the nitrogenous bases are grouped according to the number of hydrogen bonds and the number of condensed rings contained in the molecular structures of nitrogenous bases;
   - the matrix is preformed by raising tensors of each previous-generation matrix to the second power multiple times in order to obtain a next-generation matrix;
   - wherein the system binary indices that form the i data bits, correspond to the selected characteristic of the nitrogenous bases;
   - the information to be coded is recorded together with a) the value n, whereas the size of the matrix to be used for the coding depends on this value; b) the information about the selected method of transforming the matrix into a sequence; c) the information reading order.

2. The method of claim 1, ***wherein*** the information to be coded is arranged as a machine-readable sequence in binary form, including binary indices for each multiplet.

3. The method of claim 1, ***wherein*** the information to be coded is arranged as a sequence of nitrogenous bases.

4. The method of claim 1, ***wherein*** each code combination of i data bits is supplemented by a combination of m control bits based on the combination of the i and k bits;

5. The method of claim 1, ***wherein*** one can assess the recessive or dominant trait of the corresponding logically complete chunk of information, based on the position of each multiplet in the matrix of the molecular genetic system.

6. The method of claim 1, ***wherein*** the sequence of nitrogenous bases is divided into logically complete chunks, in which the information about the beginning and the end of the chunk of information is coded.

7. The method of claim 3, ***wherein*** the information to be coded is arranged as a sequence of nitrogenous bases to be coded on the DNA level or on the RNA level or on protein level or is arranged as a sequence of amino acids.

**8.** The method of claim 1, ***wherein*** the molecular genetic system has a linear or a square or a rectangular or a circular or a volume matrix representation.

**9.** The method of claim 1, ***wherein*** the molecular genetic system has a tree structure matrix representation.

**10.** The method of claim 1, ***wherein*** the binary indices of the molecular genetic system correspond to the selected characteristic of the nitrogenous bases for DNA or for RNA or for amino acids.

**11.** The method of claim 1, ***wherein*** the binary indices of the molecular genetic system correspond to the selected characteristic of the nitrogenous bases forming the recessive and dominant traits on the genetic level.

**12.** The method of claim 1, ***wherein*** the synthetic DNA formed from the obtained sequence of nitrogenous bases and containing the information being coded is placed into a storage medium.

**13.** The method of claim 1, ***wherein*** the synthetic DNA formed from the obtained sequence of nitrogenous bases and containing the information being coded is placed into a medium for logical and mathematical calculations.

**14.** Method of decoding information, whereby

- the machine-readable sequence is divided into logically complete chunks of information to be decoded; the chunks include combinations of i data bits and k check bits; each logically complete chunk of information is associated with at least one multiplet consisting of the n number of nitrogenous bases / corresponding amino acids, and being an element of the molecular genetic system used for the decoding,

***the method comprising***

- the molecular genetic system which consists of a base representing a four-nitrogenous-base matrix $\begin{bmatrix} G & C \\ A & T/U \end{bmatrix}$; the nitrogenous bases are grouped according to the number of hydrogen bonds and the number of condensed rings contained in the molecular structures of nitrogenous bases;
- preforming the matrix by raising tensors of each previous-generation matrix to the second power multiple times in order to obtain a next-generation matrix;
- wherein the system binary indices that form the i data bits correspond to the selected characteristic of the nitrogenous bases, and the indices that form the k check bits is defined based on the combination of the i data bits.

**15.** The method of claim 14, ***wherein*** the discrete parts of the machine-readable sequence consisting of logically complete chunks of information to be decoded include combinations of m control bits and that indices that form the m control bits are defined based on the combinations of i data bits.

**16.** The method of claim 14, ***wherein*** one can assess the recessive or dominant trait of the corresponding logically complete chunk of information, based on the position of each multiplet in the matrix of the molecular genetic system.

**17.** The method of claim 14, ***wherein*** the molecular genetic system has a linear or a square or a rectangular or a circular or a volume matrix representation.

**18.** The method of claim 14, ***wherein*** the molecular genetic system has a tree structure matrix representation.

**19.** The method of claim 14, ***wherein*** the binary indices of the molecular genetic system correspond to the selected characteristic of the nitrogenous bases for DNA or for RNA or for amino acids.

**20.** The method of claim 14, ***wherein*** the binary indices of the molecular genetic system correspond to the selected characteristic of the nitrogenous bases forming the recessive and dominant traits on the genetic level.

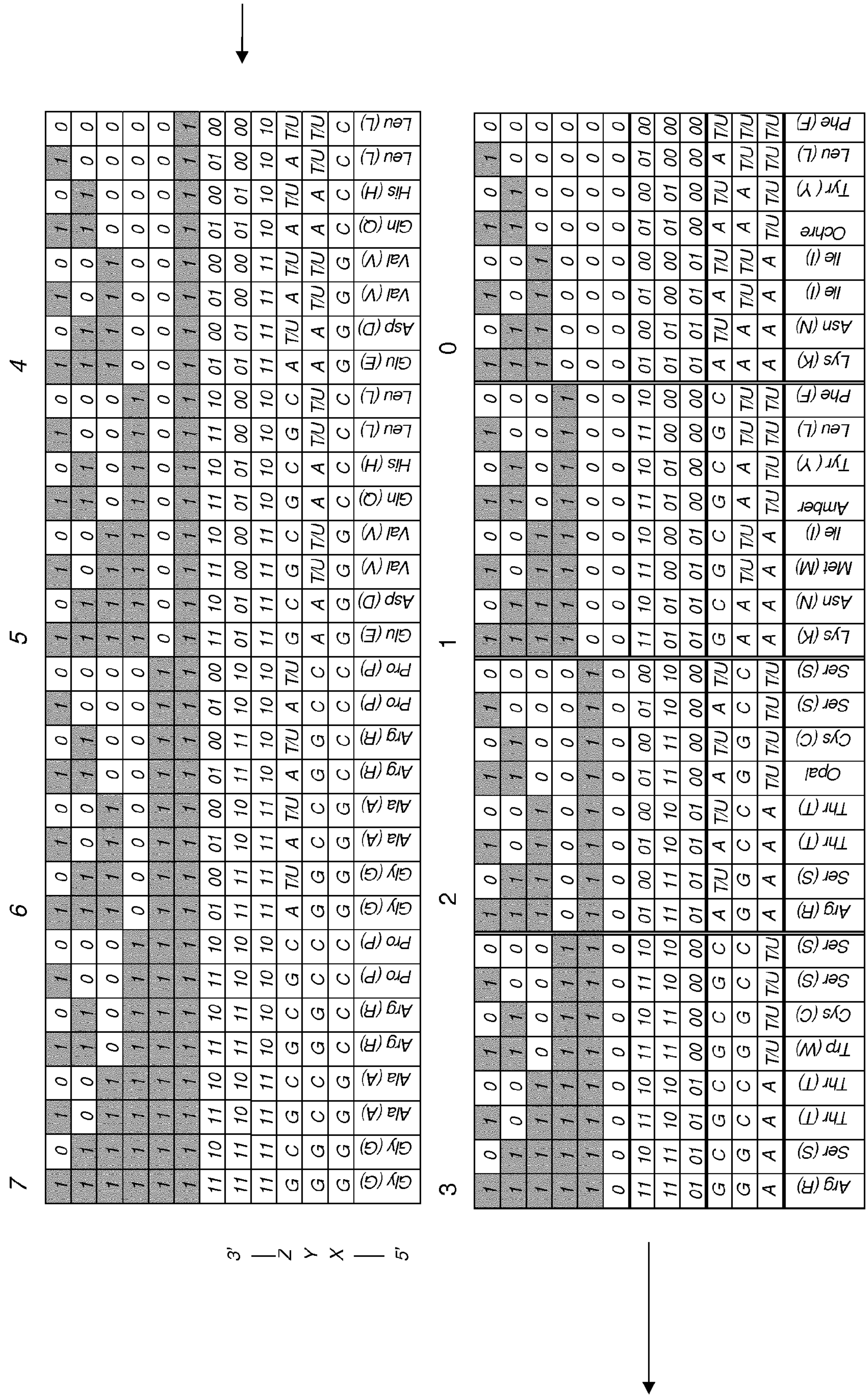

Fig. 1

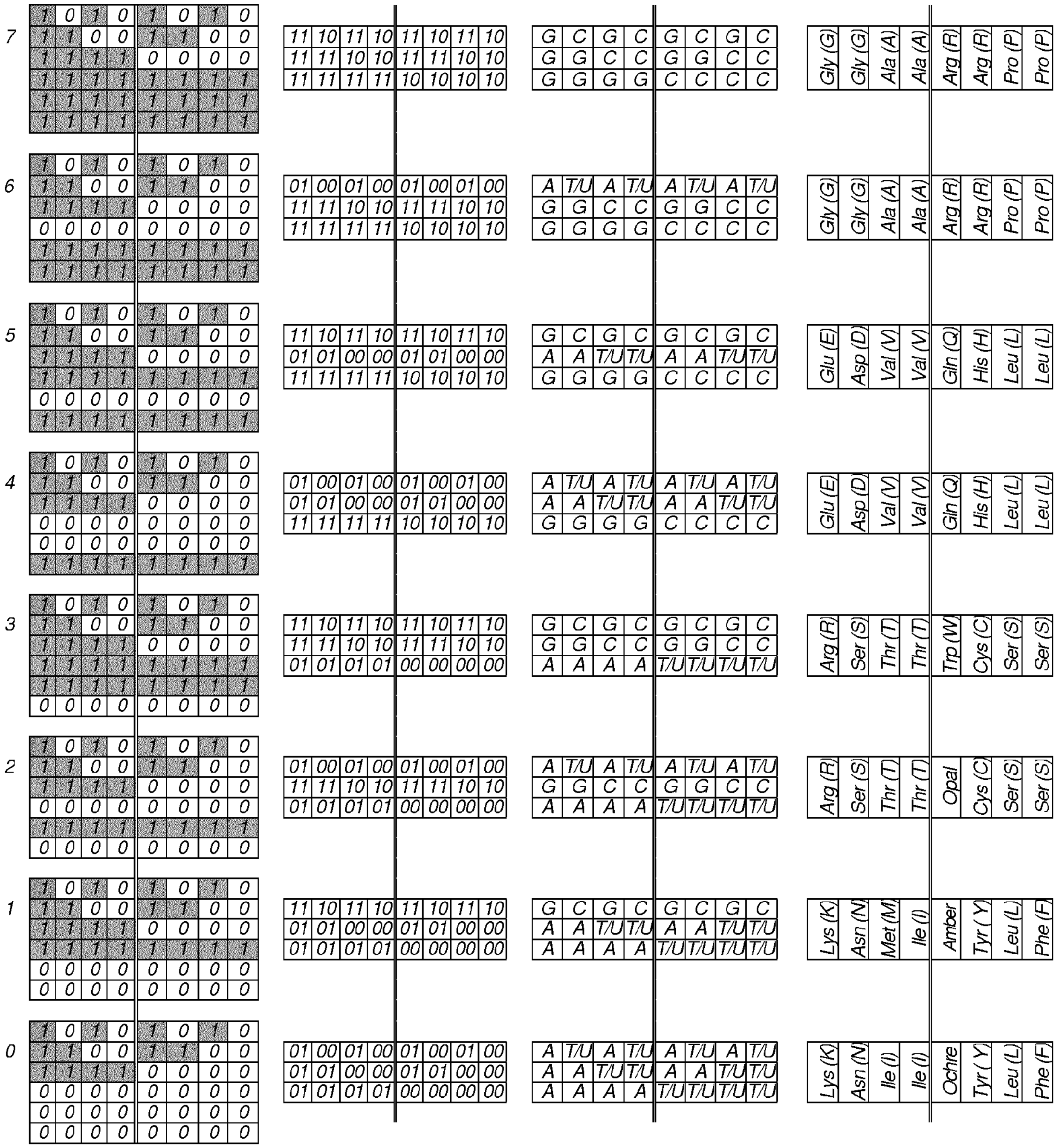
7
G C G C G C G C
G G C C G G C C
G G G G C C C C
Gly (G) Gly (G) Ala (A) Ala (A) Arg (R) Arg (R) Pro (P) Pro (P)
6
A T/U A T/U A T/U A T/U
G G C C G G C C
G G G G C C C C
Gly (G) Gly (G) Ala (A) Ala (A) Arg (R) Arg (R) Pro (P) Pro (P)
5
G C G C G C G C
A A T/U T/U A A T/U T/U
G G G G C C C C
Glu (E) Asp (D) Val (V) Val (V) Gln (Q) His (H) Leu (L) Leu (L)
4
A T/U A T/U A T/U A T/U
A A T/U T/U A A T/U T/U
G G G G C C C C
Glu (E) Asp (D) Val (V) Val (V) Gln (Q) His (H) Leu (L) Leu (L)
3
G C G C G C G C
G G C C G G C C
A A A A T/U T/U T/U T/U
Arg (R) Ser (S) Thr (T) Thr (T) Trp (W) Cys (C) Ser (S) Ser (S)
2
A T/U A T/U A T/U A T/U
G G C C G G C C
A A A A T/U T/U T/U T/U
Arg (R) Ser (S) Thr (T) Thr (T) Opal Cys (C) Ser (S) Ser (S)
1
G C G C G C G C
A A T/U T/U A A T/U T/U
A A A A T/U T/U T/U T/U
Lys (K) Asn (N) Met (M) Ile (I) Amber Tyr (Y) Leu (L) Phe (F)
0
A T/U A T/U A T/U A T/U
A A T/U T/U A A T/U T/U
A A A A T/U T/U T/U T/U
Lys (K) Asn (N) Ile (I) Ile (I) Ochre Tyr (Y) Leu (L) Phe (F)

Fig. 2

| 3 | | | | | | | |
|---|---|---|---|---|---|---|---|
| 1 | 0 | 1 | 0 | 1 | 0 | 1 | 0 |
| 1 | 1 | 0 | 0 | 1 | 1 | 0 | 0 |
| 1 | 1 | 1 | 1 | 0 | 0 | 0 | 0 |
| 1 | 1 | 1 | 1 | 1 | 1 | 1 | 1 |
| 1 | 1 | 1 | 1 | 1 | 1 | 1 | 1 |
| 0 | 0 | 0 | 0 | 0 | 0 | 0 | 0 |
| 11 | 10 | 11 | 10 | 11 | 10 | 11 | 10 |
| 11 | 11 | 10 | 10 | 11 | 11 | 10 | 10 |
| 01 | 01 | 01 | 01 | 00 | 00 | 00 | 00 |
| G | C | G | C | G | C | G | C |
| G | G | C | C | G | G | C | C |
| A | A | A | A | T/U | T/U | T/U | T/U |
| Arg (R) | Ser (S) | Thr (T) | Thr (T) | Trp (W) | Cys (C) | Ser (S) | Ser (S) |

| 2 | | | | | | | |
|---|---|---|---|---|---|---|---|
| 1 | 0 | 1 | 0 | 1 | 0 | 1 | 0 |
| 1 | 1 | 0 | 0 | 1 | 1 | 0 | 0 |
| 1 | 1 | 1 | 1 | 0 | 0 | 0 | 0 |
| 0 | 0 | 0 | 0 | 0 | 0 | 0 | 0 |
| 1 | 1 | 1 | 1 | 1 | 1 | 1 | 1 |
| 0 | 0 | 0 | 0 | 0 | 0 | 0 | 0 |
| 01 | 00 | 01 | 00 | 01 | 00 | 01 | 00 |
| 11 | 11 | 10 | 10 | 11 | 11 | 10 | 10 |
| 01 | 01 | 01 | 01 | 00 | 00 | 00 | 00 |
| A | T/U | A | T/U | A | T/U | A | T/U |
| G | G | C | C | G | G | C | C |
| A | A | A | A | T/U | T/U | T/U | T/U |
| Arg (R) | Ser (S) | Thr (T) | Thr (T) | Opal | Cys (C) | Ser (S) | Ser (S) |

| 1 | | | | | | | |
|---|---|---|---|---|---|---|---|
| 1 | 0 | 1 | 0 | 1 | 0 | 1 | 0 |
| 1 | 1 | 0 | 0 | 1 | 1 | 0 | 0 |
| 1 | 1 | 1 | 1 | 0 | 0 | 0 | 0 |
| 1 | 1 | 1 | 1 | 1 | 1 | 1 | 1 |
| 0 | 0 | 0 | 0 | 0 | 0 | 0 | 0 |
| 0 | 0 | 0 | 0 | 0 | 0 | 0 | 0 |
| 11 | 10 | 11 | 10 | 11 | 10 | 11 | 10 |
| 01 | 01 | 00 | 00 | 01 | 01 | 00 | 00 |
| 01 | 01 | 01 | 01 | 00 | 00 | 00 | 00 |
| G | C | G | C | G | C | G | C |
| A | A | T/U | T/U | A | A | T/U | T/U |
| A | A | A | A | T/U | T/U | T/U | T/U |
| Lys (K) | Asn (N) | Met (M) | Ile (I) | Amber | Tyr (Y) | Leu (L) | Phe (F) |

| 0 | | | | | | | |
|---|---|---|---|---|---|---|---|
| 1 | 0 | 1 | 0 | 1 | 0 | 1 | 0 |
| 1 | 1 | 0 | 0 | 1 | 1 | 0 | 0 |
| 1 | 1 | 1 | 1 | 0 | 0 | 0 | 0 |
| 0 | 0 | 0 | 0 | 0 | 0 | 0 | 0 |
| 0 | 0 | 0 | 0 | 0 | 0 | 0 | 0 |
| 0 | 0 | 0 | 0 | 0 | 0 | 0 | 0 |
| 01 | 00 | 01 | 00 | 01 | 00 | 01 | 00 |
| 01 | 01 | 00 | 00 | 01 | 01 | 00 | 00 |
| 01 | 01 | 01 | 01 | 00 | 00 | 00 | 00 |
| A | T/U | A | T/U | A | T/U | A | T/U |
| A | A | T/U | T/U | A | A | T/U | T/U |
| A | A | A | A | T/U | T/U | T/U | T/U |
| Lys (K) | Asn (N) | Ile (I) | Ile (I) | Ochre | Tyr (Y) | Leu (L) | Phe (F) |

| | 4 | | | | | | | |
|---|---|---|---|---|---|---|---|---|
| | 0 | 1 | 0 | 1 | 0 | 1 | 0 | 1 |
| | 0 | 0 | 1 | 1 | 0 | 0 | 1 | 1 |
| | 0 | 0 | 0 | 0 | 1 | 1 | 1 | 1 |
| | 0 | 0 | 0 | 0 | 0 | 0 | 0 | 0 |
| | 0 | 0 | 0 | 0 | 0 | 0 | 0 | 0 |
| | 1 | 1 | 1 | 1 | 1 | 1 | 1 | 1 |
| | 00 | 01 | 00 | 01 | 00 | 01 | 00 | 01 |
| | 00 | 00 | 01 | 01 | 00 | 00 | 01 | 01 |
| | 10 | 10 | 10 | 10 | 11 | 11 | 11 | 11 |
| | T/U | A | T/U | A | T/U | A | T/U | A |
| | T/U | T/U | A | A | T/U | T/U | A | A |
| | C | C | C | C | G | G | G | G |
| | Leu (L) | Leu (L) | His (H) | Gln (Q) | Val (V) | Val (V) | Asp (D) | Glu (E) |
| Σ (number of hydrogen bonds, columns) | 15 | 15 | 15 | 15 | 15 | 15 | 15 | 15 |

| 5 | | | | | | | |
|---|---|---|---|---|---|---|---|
| 0 | 1 | 0 | 1 | 0 | 1 | 0 | 1 |
| 0 | 0 | 1 | 1 | 0 | 0 | 1 | 1 |
| 0 | 0 | 0 | 0 | 1 | 1 | 1 | 1 |
| 1 | 1 | 1 | 1 | 1 | 1 | 1 | 1 |
| 0 | 0 | 0 | 0 | 0 | 0 | 0 | 0 |
| 1 | 1 | 1 | 1 | 1 | 1 | 1 | 1 |
| 10 | 11 | 10 | 11 | 10 | 11 | 10 | 11 |
| 00 | 00 | 01 | 01 | 00 | 00 | 01 | 01 |
| 10 | 10 | 10 | 10 | 11 | 11 | 11 | 11 |
| C | G | C | G | C | G | C | G |
| T/U | T/U | A | A | T/U | T/U | A | A |
| C | C | C | C | G | G | G | G |
| Leu (L) | Leu (L) | His (H) | Gln (Q) | Val (V) | Val (V) | Asp (D) | Glu (E) |
| 15 | 15 | 15 | 15 | 15 | 15 | 15 | 15 |

| 6 | | | | | | | |
|---|---|---|---|---|---|---|---|
| 0 | 1 | 0 | 1 | 0 | 1 | 0 | 1 |
| 0 | 0 | 1 | 1 | 0 | 0 | 1 | 1 |
| 0 | 0 | 0 | 0 | 1 | 1 | 1 | 1 |
| 0 | 0 | 0 | 0 | 0 | 0 | 0 | 0 |
| 1 | 1 | 1 | 1 | 1 | 1 | 1 | 1 |
| 1 | 1 | 1 | 1 | 1 | 1 | 1 | 1 |
| 00 | 01 | 00 | 01 | 00 | 01 | 00 | 01 |
| 10 | 10 | 11 | 11 | 10 | 10 | 11 | 11 |
| 10 | 10 | 10 | 10 | 11 | 11 | 11 | 11 |
| T/U | A | T/U | A | T/U | A | T/U | A |
| C | C | G | G | C | C | G | G |
| C | C | C | C | G | G | G | G |
| Pro (P) | Pro (P) | Arg (R) | Arg (R) | Ala (A) | Ala (A) | Gly (G) | Gly (G) |
| 15 | 15 | 15 | 15 | 15 | 15 | 15 | 15 |

| 7 | | | | | | | | |
|---|---|---|---|---|---|---|---|---|
| 0 | 1 | 0 | 1 | 0 | 1 | 0 | 1 | Σ (number of hydrogen bonds, lines) |
| 0 | 0 | 1 | 1 | 0 | 0 | 1 | 1 | |
| 0 | 0 | 0 | 0 | 1 | 1 | 1 | 1 | |
| 1 | 1 | 1 | 1 | 1 | 1 | 1 | 1 | |
| 1 | 1 | 1 | 1 | 1 | 1 | 1 | 1 | |
| 1 | 1 | 1 | 1 | 1 | 1 | 1 | 1 | |
| 10 | 11 | 10 | 11 | 10 | 11 | 10 | 11 | |
| 10 | 10 | 11 | 11 | 10 | 10 | 11 | 11 | |
| 10 | 10 | 10 | 10 | 11 | 11 | 11 | 11 | |
| C | G | C | G | C | G | C | G | 160 |
| C | C | G | G | C | C | G | G | 160 |
| C | C | C | C | G | G | G | G | 160 |
| Pro (P) | Pro (P) | Arg (R) | Arg (R) | Ala (A) | Ala (A) | Gly (G) | Gly (G) | |
| 15 | 15 | 15 | 15 | 15 | 15 | 15 | 15 | |

Fig. 3

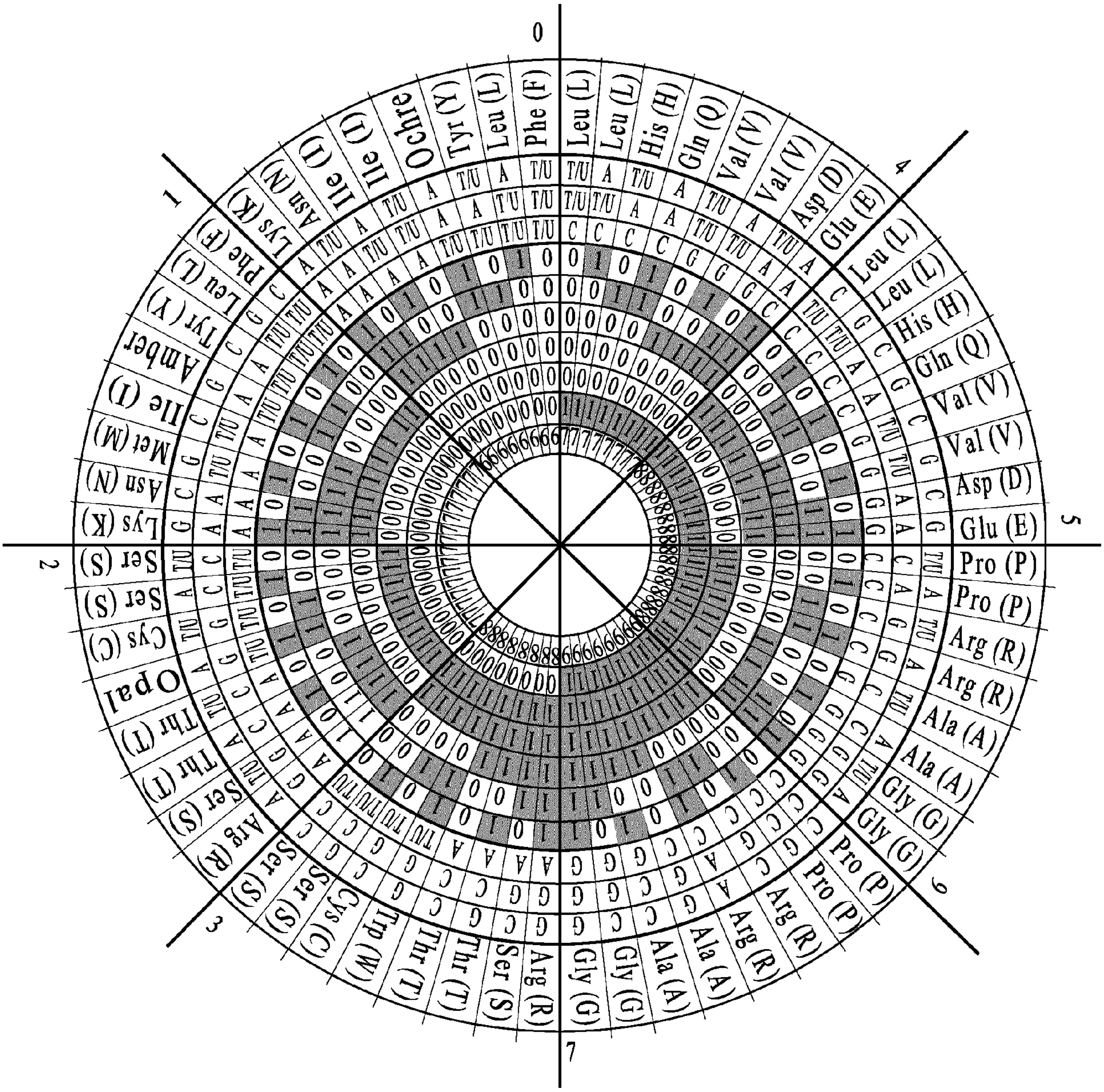
0
Leu (L)
Leu (L)
His (H)
Gln (Q)
Val (V)
Val (V)
Asp (D)
Glu (E)
4
Leu (L)
Leu (L)
His (H)
Gln (Q)
Val (V)
Val (V)
Asp (D)
Glu (E)
5
Pro (P)
Pro (P)
Arg (R)
Arg (R)
Ala (A)
Ala (A)
Gly (G)
Gly (G)
6
Pro (P)
Pro (P)
Arg (R)
Arg (R)
Ala (A)
Ala (A)
Gly (G)
Gly (G)
7
Arg (R)
Ser (S)
Thr (T)
Thr (T)
Trp (W)
Cys (C)
Ser (S)
Ser (S)
3
Arg (R)
Ser (S)
Thr (T)
Thr (T)
Opal
Cys (C)
Ser (S)
Ser (S)
2
Lys (K)
Asn (N)
Met (M)
Ile (I)
Amber
Tyr (Y)
Leu (L)
Phe (F)
1
Lys (K)
Asn (N)
Ile (I)
Ile (I)
Ochre
Tyr (Y)
Leu (L)
Phe (F)

Fig. 4

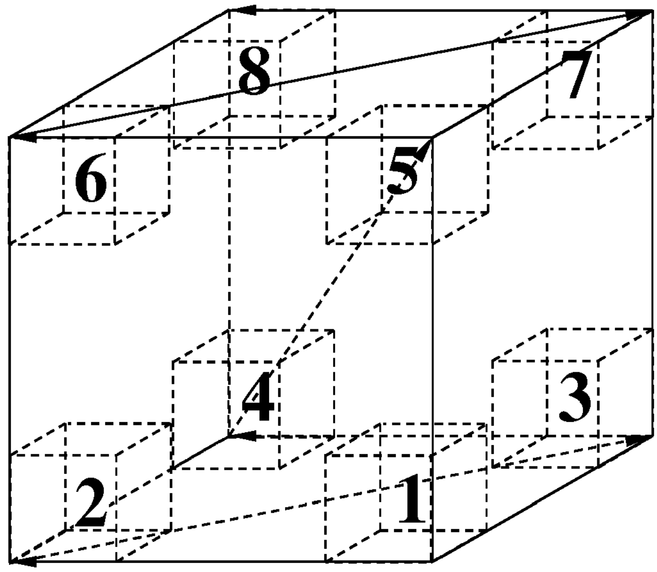


Fig. 5

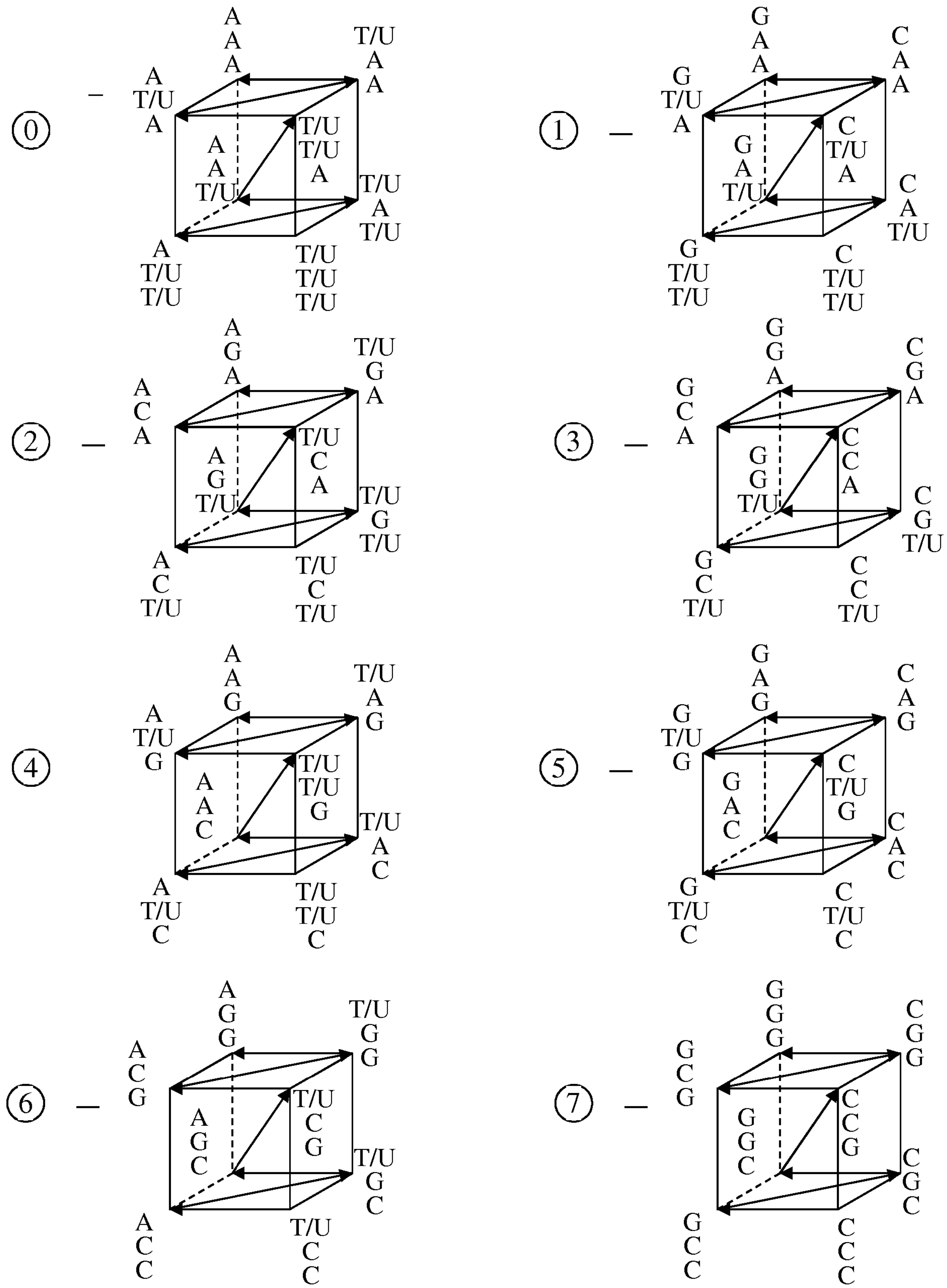
0
A A A
T/U A A
A T/U A
T/U T/U A
A A T/U
T/U A T/U
A T/U T/U
T/U T/U T/U
1
G A A
C A A
G T/U A
C T/U A
G A T/U
C A T/U
G T/U T/U
C T/U T/U
2
A G A
T/U G A
A C A
T/U C A
A G T/U
T/U G T/U
A C T/U
T/U C T/U
3
G G A
C G A
G C A
C C A
G G T/U
C G T/U
G C T/U
C C T/U
4
A A G
T/U A G
A T/U G
T/U T/U G
A A C
T/U A C
A T/U C
T/U T/U C
5
G A G
C A G
G T/U G
C T/U G
G A C
C A C
G T/U C
C T/U C
6
A G G
T/U G G
A C G
T/U C G
A G C
T/U G C
A C C
T/U C C
7
G G G
C G G
G C G
C C G
G G C
C G C
G C C
C C C

Fig. 5A

$F^3$ =

| | | | | | | | |
|---|---|---|---|---|---|---|---|
| 63 *GGG* | 62 *GGC* | 61 *GCG* | 60 *GCC* | 59 *CGG* | 58 *CGC* | 57 *CCG* | 56 *CCC* |
| 55 *GGA* | 54 *GGT* | 53 *GCA* | 52 *GCT* | 51 *CGA* | 50 *CGT* | 49 *CCA* | 48 *CCT* |
| 47 *GAG* | 46 *GAC* | 45 *GTG* | 44 *GTC* | 43 *CAG* | 42 *CAC* | 41 *CTG* | 40 *CTC* |
| 39 *GAA* | 38 *GAT* | 37 *GTA* | 36 *GTT* | 35 *CAA* | 34 *CAT* | 33 *CTA* | 32 *CTT* |
| 31 *AGG* | 30 *AGC* | 29 *ACG* | 28 *ACC* | 27 *TGG* | 26 *TGC* | 25 *TCG* | 24 *TCC* |
| 23 *AGA* | 22 *AGT* | 21 *ACA* | 20 *ACT* | 19 *TGA* | 18 *TGT* | 17 *TCA* | 16 *TCT* |
| 15 *AAG* | 14 *AAC* | 13 *ATG* | 12 *ATC* | 11 *TAG* | 10 *TAC* | 9 *TTG* | 8 *TTC* |
| 7 *AAA* | 6 *AAT* | 5 *ATA* | 4 *ATT* | 3 *TAA* | 2 *TAT* | 1 *TTA* | 0 *TTT* |

Fig. 6

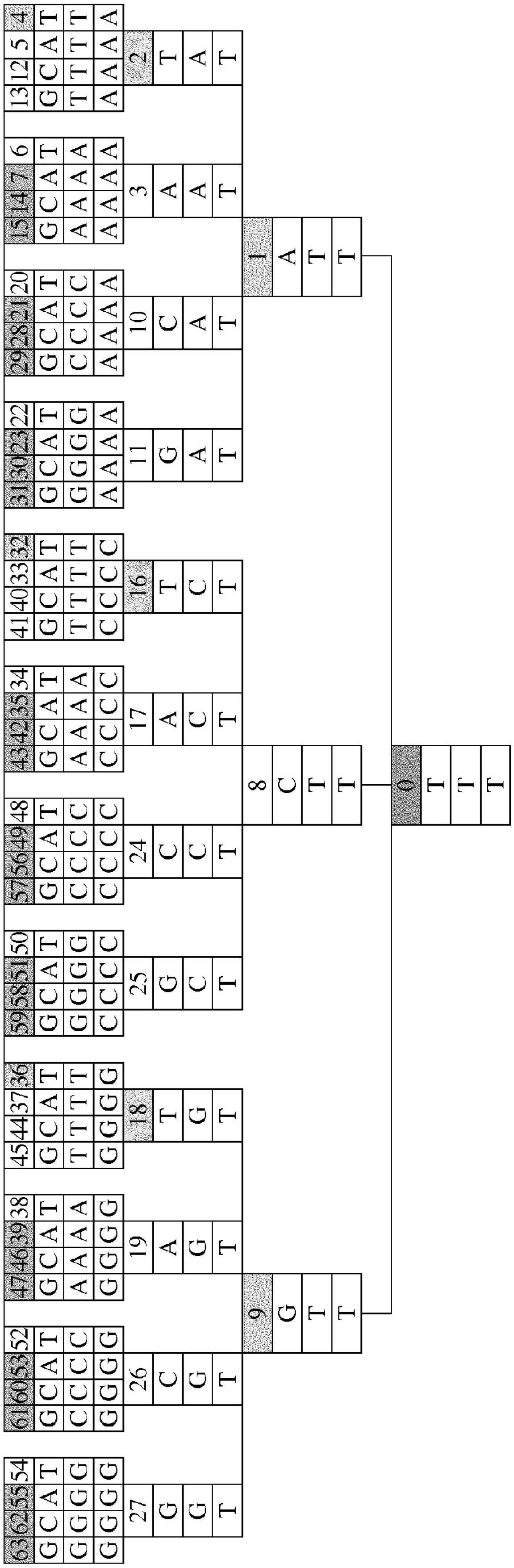

Fig. 6A

| | **7** | **6** | **5** | 4 | 3 | 2 | 1 | 0 | Σ |
|---|---|---|---|---|---|---|---|---|---|
| **7** | $G-3$ | $C-3$ | $G-3$ | $C-3$ | $G-3$ | $C-3$ | $G-3$ | $C-3$ | **24** |
| | $G-3$ | $G-3$ | $C-3$ | $C-3$ | $G-3$ | $G-3$ | $C-3$ | $C-3$ | **24** |
| | $G-3$ | $G-3$ | $G-3$ | $G-3$ | $C-3$ | $C-3$ | $C-3$ | $C-3$ | **24** |
| **6** | $A-2$ | $T/U-2$ | $A-2$ | $T/U-2$ | $A-2$ | $T/U-2$ | $A-2$ | $T/U-2$ | **16** |
| | $G-3$ | $G-3$ | $C-3$ | $C-3$ | $G-3$ | $G-3$ | $C-3$ | $C-3$ | **24** |
| | $G-3$ | $G-3$ | $G-3$ | $G-3$ | $C-3$ | $C-3$ | $C-3$ | $C-3$ | **24** |
| **5** | $G-3$ | $C-3$ | $G-3$ | $C-3$ | $G-3$ | $C-3$ | $G-3$ | $C-3$ | **24** |
| | $A-2$ | $A-2$ | $T/U-2$ | $T/U-2$ | $A-2$ | $A-2$ | $T/U-2$ | $T/U-2$ | **16** |
| | $G-3$ | $G-3$ | $G-3$ | $G-3$ | $C-3$ | $C-3$ | $C-3$ | $C-3$ | **24** |
| **4** | $A-2$ | $T/U-2$ | $A-2$ | $T/U-2$ | $A-2$ | $T/U-2$ | $A-2$ | $T/U-2$ | **16** |
| | $A-2$ | $A-2$ | $T/U-2$ | $T/U-2$ | $A-2$ | $A-2$ | $T/U-2$ | $T/U-2$ | **16** |
| | $G-3$ | $G-3$ | $G-3$ | $G-3$ | $C-3$ | $C-3$ | $C-3$ | $C-3$ | **24** |
| **3** | $G-3$ | $C-3$ | $G-3$ | $C-3$ | $G-3$ | $C-3$ | $G-3$ | $C-3$ | **24** |
| | $G-3$ | $G-3$ | $C-3$ | $C-3$ | $G-3$ | $G-3$ | $C-3$ | $C-3$ | **24** |
| | $A-2$ | $A-2$ | $A-2$ | $A-2$ | $T/U-2$ | $T/U-2$ | $T/U-2$ | $T/U-2$ | **16** |
| **2** | $A-2$ | $T/U-2$ | $A-2$ | $T/U-2$ | $A-2$ | $T/U-2$ | $A-2$ | $T/U-2$ | **16** |
| | $G-3$ | $G-3$ | $C-3$ | $C-3$ | $G-3$ | $G-3$ | $C-3$ | $C-3$ | **24** |
| | $A-2$ | $A-2$ | $A-2$ | $A-2$ | $T/U-2$ | $T/U-2$ | $T/U-2$ | $T/U-2$ | **16** |
| **1** | $G-3$ | $C-3$ | $G-3$ | $C-3$ | $G-3$ | $C-3$ | $G-3$ | $C-3$ | **24** |
| | $A-2$ | $A-2$ | $T/U-2$ | $T/U-2$ | $A-2$ | $A-2$ | $T/U-2$ | $T/U-2$ | **16** |
| | $A-2$ | $A-2$ | $A-2$ | $A-2$ | $T/U-2$ | $T/U-2$ | $T/U-2$ | $T/U-2$ | **16** |
| **0** | $A-2$ | $T/U-2$ | $A-2$ | $T/U-2$ | $A-2$ | $T/U-2$ | $A-2$ | $T/U-2$ | **16** |
| | $A-2$ | $A-2$ | $T/U-2$ | $T/U-2$ | $A-2$ | $A-2$ | $T/U-2$ | $T/U-2$ | **16** |
| | $A-2$ | $A-2$ | $A-2$ | $A-2$ | $T/U-2$ | $T/U-2$ | $T/U-2$ | $T/U-2$ | **16** |
| **Σ** | **60** | **60** | **60** | **60** | **60** | **60** | **60** | **60** | **480** |

Fig. 8

| 7 | | | | | | | | 6 | | | | | | | | 5 | | | | | | | | 4 | | | | | | | | 3 | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|
| G 3 | C 3 | G 3 | C 3 | G 3 | C 3 | G 3 | C 3 | A 2 | T 2 | A 2 | T 2 | A 2 | T 2 | A 2 | T 2 | G 3 | C 3 | G 3 | C 3 | G 3 | C 3 | G 3 | C 3 | A 2 | T 2 | A 2 | T 2 | A 2 | T 2 | A 2 | T 2 | G 3 | C 3 | G 3 | C 3 | G 3 | C 3 | G 3 | C 3 | |
| G 3 | G 3 | C 3 | C 3 | G 3 | G 3 | C 3 | C 3 | G 3 | G 3 | C 3 | C 3 | G 3 | G 3 | C 3 | C 3 | A 2 | A 2 | T 2 | T 2 | A 2 | A 2 | T 2 | T 2 | A 2 | A 2 | T 2 | T 2 | A 2 | A 2 | T 2 | T 2 | G 3 | G 3 | C 3 | C 3 | G 3 | G 3 | C 3 | C 3 | → |
| G 3 | G 3 | G 3 | G 3 | C 3 | C 3 | C 3 | C 3 | G 3 | G 3 | G 3 | G 3 | C 3 | C 3 | C 3 | C 3 | G 3 | G 3 | G 3 | G 3 | C 3 | C 3 | C 3 | C 3 | G 3 | G 3 | G 3 | G 3 | C 3 | C 3 | C 3 | C 3 | A 2 | A 2 | A 2 | A 2 | T 2 | T 2 | T 2 | T 2 | |
| **9** | **9** | **9** | **9** | **9** | **9** | **9** | **9** | **8** | **8** | **8** | **8** | **8** | **8** | **8** | **8** | **8** | **8** | **8** | **8** | **8** | **8** | **8** | **8** | **7** | **7** | **7** | **7** | **7** | **7** | **7** | **7** | **8** | **8** | **8** | **8** | **8** | **8** | **8** | **8** | |

| | 2 | | | | | | | | 1 | | | | | | | | 0 | | | | | | | | total 7 | 6 | 5 | 4 | 3 | 2 | 1 | 0 | Σ |
|---|---|---|---|---|---|---|---|---|---|---|---|---|---|---|---|---|---|---|---|---|---|---|---|---|---|---|---|---|---|---|---|---|---|
| | A 2 | T 2 | A 2 | T 2 | A 2 | T 2 | A 2 | T 2 | G 3 | C 3 | G 3 | C 3 | G 3 | C 3 | G 3 | C 3 | A 2 | T 2 | A 2 | T 2 | A 2 | T 2 | A 2 | T 2 | 24 | 16 | 24 | 16 | 24 | 16 | 24 | 16 | 160 |
| → | G 3 | G 3 | C 3 | C 3 | G 3 | G 3 | C 3 | C 3 | A 2 | A 2 | T 2 | T 2 | A 2 | A 2 | T 2 | T 2 | A 2 | A 2 | T 2 | T 2 | A 2 | A 2 | T 2 | T 2 | 24 | 24 | 16 | 16 | 24 | 24 | 16 | 16 | 160 |
| | A 2 | A 2 | A 2 | A 2 | T 2 | T 2 | T 2 | T 2 | A 2 | A 2 | A 2 | A 2 | T 2 | T 2 | T 2 | T 2 | A 2 | A 2 | A 2 | A 2 | T 2 | T 2 | T 2 | T 2 | 24 | 24 | 24 | 24 | 16 | 16 | 16 | 16 | 160 |
| | **7** | **7** | **7** | **7** | **7** | **7** | **7** | **7** | **7** | **7** | **7** | **7** | **7** | **7** | **7** | **7** | **6** | **6** | **6** | **6** | **6** | **6** | **6** | **6** | **72** | **64** | **64** | **56** | **64** | **56** | **56** | **48** | **480** |

Fig. 7

| 7 | | | | | | | | 6 | | | | | | | |
|---|---|---|---|---|---|---|---|---|---|---|---|---|---|---|---|
| G | C | G | C | G | C | G | C | A | T | A | T | A | T | A | T |
| 16 | 13 | 16 | 13 | 16 | 13 | 16 | 13 | 15 | 15 | 15 | 15 | 15 | 15 | 15 | 15 |
| G | G | C | C | G | G | C | C | G | G | C | C | G | G | C | C |
| 16 | 16 | 13 | 13 | 16 | 16 | 13 | 13 | 16 | 16 | 13 | 13 | 16 | 16 | 13 | 13 |
| G | G | G | G | C | C | C | C | G | G | G | G | C | C | C | C |
| 16 | 16 | 16 | 16 | 13 | 13 | 13 | 13 | 16 | 16 | 16 | 16 | 13 | 13 | 13 | 13 |
| **48** | **45** | **45** | **42** | **45** | **42** | **42** | **39** | **47** | **47** | **44** | **44** | **44** | **44** | **41** | **41** |

| 5 | | | | | | | | 4 | | | | | | | | 3 | | | | | | | | |
|---|---|---|---|---|---|---|---|---|---|---|---|---|---|---|---|---|---|---|---|---|---|---|---|---|
| G | C | G | C | G | C | G | C | A | T | A | T | A | T | A | T | G | C | G | C | G | C | G | C | |
| 16 | 13 | 16 | 13 | 16 | 13 | 16 | 13 | 15 | 15 | 15 | 15 | 15 | 15 | 15 | 15 | 16 | 13 | 16 | 13 | 16 | 13 | 16 | 13 | |
| A | A | T | T | A | A | T | T | A | A | T | T | A | A | T | T | G | G | C | C | G | G | C | C | → |
| 15 | 15 | 15 | 15 | 15 | 15 | 15 | 15 | 15 | 15 | 15 | 15 | 15 | 15 | 15 | 15 | 16 | 16 | 13 | 13 | 16 | 16 | 13 | 13 | |
| G | G | G | G | C | C | C | C | G | G | G | G | C | C | C | C | A | A | A | A | T | T | T | T | |
| 16 | 16 | 16 | 16 | 13 | 13 | 13 | 13 | 16 | 16 | 16 | 16 | 13 | 13 | 13 | 13 | 15 | 15 | 15 | 15 | 15 | 15 | 15 | 15 | |
| **47** | **44** | **47** | **44** | **44** | **41** | **44** | **41** | **46** | **46** | **46** | **46** | **43** | **43** | **43** | **43** | **47** | **44** | **44** | **41** | **47** | **44** | **44** | **41** | |

| | 2 | | | | | | | | 1 | | | | | | | |
|---|---|---|---|---|---|---|---|---|---|---|---|---|---|---|---|---|
| | A | T | A | T | A | T | A | T | G | C | G | C | G | C | G | C |
| | 15 | 15 | 15 | 15 | 15 | 15 | 15 | 15 | 16 | 13 | 16 | 13 | 16 | 13 | 16 | 13 |
| → | G | G | C | C | G | G | C | C | A | A | T | T | A | A | T | T |
| | 16 | 16 | 13 | 13 | 16 | 16 | 13 | 13 | 15 | 15 | 15 | 15 | 15 | 15 | 15 | 15 |
| | A | A | A | A | T | T | T | T | A | A | A | A | T | T | T | T |
| | 15 | 15 | 15 | 15 | 15 | 15 | 15 | 15 | 15 | 15 | 15 | 15 | 15 | 15 | 15 | 15 |
| | **46** | **46** | **43** | **43** | **46** | **46** | **43** | **43** | **46** | **43** | **46** | **43** | **46** | **43** | **46** | **43** |

| 0 | | | | | | | | total 7 | 6 | 5 | 4 | 3 | 2 | 1 | 0 | Σ |
|---|---|---|---|---|---|---|---|---|---|---|---|---|---|---|---|---|
| A | T | A | T | A | T | A | T | | | | | | | | | |
| 15 | 15 | 15 | 15 | 15 | 15 | 15 | 15 | 116 | 120 | 116 | 120 | 116 | 120 | 116 | 120 | **304** |
| A | A | T | T | A | A | T | T | | | | | | | | | |
| 15 | 15 | 15 | 15 | 15 | 15 | 15 | 15 | 116 | 116 | 120 | 120 | 116 | 116 | 120 | 120 | **304** |
| A | A | A | A | T | T | T | T | | | | | | | | | |
| 15 | 15 | 15 | 15 | 15 | 15 | 15 | 15 | 116 | 116 | 116 | 116 | 120 | 120 | 120 | 120 | **304** |
| **45** | **45** | **45** | **45** | **45** | **45** | **45** | **45** | **348** | **352** | **352** | **356** | **352** | **356** | **356** | **360** | **912** |

Fig. 9

| 7 | | | | | | | |
|---|---|---|---|---|---|---|---|
| G 5 | C 4 | G 5 | C 4 | G 5 | C 4 | G 5 | C 4 |
| G 5 | G 5 | C 4 | C 4 | G 5 | G 5 | C 4 | C 4 |
| G 5 | G 5 | G 5 | G 5 | C 4 | C 4 | C 4 | C 4 |
| 15 | 14 | 14 | 13 | 14 | 13 | 13 | 12 |

| 6 | | | | | | | |
|---|---|---|---|---|---|---|---|
| A 5 | T 5 | A 5 | T 5 | A 5 | T 5 | A 5 | T 5 |
| G 5 | G 5 | C 4 | C 4 | G 5 | G 5 | C 4 | C 4 |
| G 5 | G 5 | G 5 | G 5 | C 4 | C 4 | C 4 | C 4 |
| 15 | 15 | 14 | 14 | 14 | 14 | 13 | 13 |

| 5 | | | | | | | |
|---|---|---|---|---|---|---|---|
| G 5 | C 4 | G 5 | C 4 | G 5 | C 4 | G 5 | C 4 |
| A 5 | A 5 | T 5 | T 5 | A 5 | A 5 | T 5 | T 5 |
| G 5 | G 5 | G 5 | G 5 | C 4 | C 4 | C 4 | C 4 |
| 15 | 14 | 15 | 14 | 14 | 13 | 14 | 13 |

| 4 | | | | | | | |
|---|---|---|---|---|---|---|---|
| A 5 | T 5 | A 5 | T 5 | A 5 | T 5 | A 5 | T 5 |
| A 5 | A 5 | T 5 | T 5 | A 5 | A 5 | T 5 | T 5 |
| G 5 | G 5 | G 5 | G 5 | C 4 | C 4 | C 4 | C 4 |
| 15 | 15 | 15 | 15 | 14 | 14 | 14 | 14 |

| 3 | | | | | | | |
|---|---|---|---|---|---|---|---|
| G 5 | C 4 | G 5 | C 4 | G 5 | C 4 | G 5 | C 4 |
| G 5 | G 5 | C 4 | C 4 | G 5 | G 5 | C 4 | C 4 |
| A 5 | A 5 | A 5 | A 5 | T 5 | T 5 | T 5 | T 5 |
| 15 | 14 | 14 | 13 | 15 | 14 | 14 | 13 |

| 2 | | | | | | | |
|---|---|---|---|---|---|---|---|
| A 5 | T 5 | A 5 | T 5 | A 5 | T 5 | A 5 | T 5 |
| G 5 | G 5 | C 4 | C 4 | G 5 | G 5 | C 4 | C 4 |
| A 5 | A 5 | A 5 | A 5 | T 5 | T 5 | T 5 | T 5 |
| 15 | 15 | 14 | 14 | 15 | 15 | 14 | 14 |

| 1 | | | | | | | |
|---|---|---|---|---|---|---|---|
| G 5 | C 4 | G 5 | C 4 | G 5 | C 4 | G 5 | C 4 |
| A 5 | A 5 | T 5 | T 5 | A 5 | A 5 | T 5 | T 5 |
| A 5 | A 5 | A 5 | A 5 | T 5 | T 5 | T 5 | T 5 |
| 15 | 14 | 15 | 14 | 15 | 14 | 15 | 14 |

| 0 | | | | | | | |
|---|---|---|---|---|---|---|---|
| A 5 | T 5 | A 5 | T 5 | A 5 | T 5 | A 5 | T 5 |
| A 5 | A 5 | T 5 | T 5 | A 5 | A 5 | T 5 | T 5 |
| A 5 | A 5 | A 5 | A 5 | T 5 | T 5 | T 5 | T 5 |
| 15 | 15 | 15 | 15 | 15 | 15 | 15 | 15 |

| 7 | 6 | 5 | 4 | 3 | 2 | 1 | 0 | Σ |
|---|---|---|---|---|---|---|---|---|
| 36 | 40 | 36 | 40 | 36 | 40 | 36 | 40 | **944** |
| 36 | 36 | 40 | 40 | 36 | 36 | 40 | 40 | **944** |
| 36 | 36 | 36 | 36 | 40 | 40 | 40 | 40 | **944** |
| **108** | **112** | **112** | **116** | **112** | **116** | **116** | **120** | **2832** |

Fig. 10

| 7 | | | | | | | | 6 | | | | | | | | 5 | | | | | | | | 4 | | | | | | | | 3 | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|
| G 5 | C 4 | G 5 | C 4 | G 5 | C 4 | G 5 | C 4 | A 5 | U 4 | A 5 | U 4 | A 5 | U 4 | A 5 | U 4 | G 5 | C 4 | G 5 | C 4 | G 5 | C 4 | G 5 | C 4 | A 5 | U 4 | A 5 | U 4 | A 5 | U 4 | A 5 | U 4 | G 5 | C 4 | G 5 | C 4 | G 5 | C 4 | G 5 | C 4 |
| G 5 | G 5 | C 4 | C 4 | G 5 | G 5 | C 4 | C 4 | G 5 | G 5 | C 4 | C 4 | G 5 | G 5 | C 4 | C 4 | A 5 | A 5 | U 4 | U 4 | A 5 | A 5 | U 4 | U 4 | A 5 | A 5 | U 4 | U 4 | A 5 | A 5 | U 4 | U 4 | G 5 | G 5 | C 4 | C 4 | G 5 | G 5 | C 4 | C 4 |
| G 5 | G 5 | G 5 | G 5 | C 4 | C 4 | C 4 | C 4 | G 5 | G 5 | G 5 | G 5 | C 4 | C 4 | C 4 | C 4 | G 5 | G 5 | G 5 | G 5 | C 4 | C 4 | C 4 | C 4 | G 5 | G 5 | G 5 | G 5 | C 4 | C 4 | C 4 | C 4 | A 5 | A 5 | A 5 | A 5 | U 4 | U 4 | U 4 | U 4 |
| 15 | 14 | 14 | 13 | 14 | 13 | 13 | 12 | 15 | 14 | 14 | 13 | 14 | 13 | 13 | 12 | 15 | 14 | 14 | 13 | 14 | 13 | 13 | 12 | 15 | 14 | 14 | 13 | 14 | 13 | 13 | 12 | 15 | 14 | 14 | 13 | 14 | 13 | 13 | 12 |

| 2 | | | | | | | | 1 | | | | | | | | 0 | | | | | | | | 7 | 6 | 5 | 4 | 3 | 2 | 1 | 0 | Σ |
|---|---|---|---|---|---|---|---|---|---|---|---|---|---|---|---|---|---|---|---|---|---|---|---|---|---|---|---|---|---|---|---|---|
| A 5 | U 4 | A 5 | U 4 | A 5 | U 4 | A 5 | U 4 | G 5 | C 4 | G 5 | C 4 | G 5 | C 4 | G 5 | C 4 | A 5 | U 4 | A 5 | U 4 | A 5 | U 4 | A 5 | U 4 | 36 | 36 | 36 | 36 | 36 | 36 | 36 | 36 | **288** |
| G 5 | G 5 | C 4 | C 4 | G 5 | G 5 | C 4 | C 4 | A 5 | A 5 | U 4 | U 4 | A 5 | A 5 | U 4 | U 4 | A 5 | A 5 | U 4 | U 4 | A 5 | A 5 | U 4 | U 4 | 36 | 36 | 36 | 36 | 36 | 36 | 36 | 36 | **288** |
| A 5 | A 5 | A 5 | A 5 | U 4 | U 4 | U 4 | U 4 | A 5 | A 5 | A 5 | A 5 | U 4 | U 4 | U 4 | U 4 | A 5 | A 5 | A 5 | A 5 | U 4 | U 4 | U 4 | U 4 | 36 | 36 | 36 | 36 | 36 | 36 | 36 | 36 | **288** |
| 15 | 14 | 14 | 13 | 14 | 13 | 13 | 12 | 15 | 14 | 14 | 13 | 14 | 13 | 13 | 12 | 15 | 14 | 14 | 13 | 14 | 13 | 13 | 12 | **108** | **108** | **108** | **108** | **108** | **108** | **108** | **108** | **864** |

Fig. 11

| 7 | | | | | | | | 6 | | | | | | | | 5 | | | | | | | | 4 | | | | | | | | 3 | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|
| G 1 | C 1 | G 1 | C 1 | G 1 | C 1 | G 1 | C 1 | A 1 | T 1 | A 1 | T 1 | A 1 | T 1 | A 1 | T 1 | G 1 | C 1 | G 1 | C 1 | G 1 | C 1 | G 1 | C 1 | A 1 | T 1 | A 1 | T 1 | A 1 | T 1 | A 1 | T 1 | G 1 | C 1 | G 1 | C 1 | G 1 | C 1 | G 1 | C 1 |
| G 1 | G 1 | C 1 | C 1 | G 1 | G 1 | C 1 | C 1 | G 1 | G 1 | C 1 | C 1 | G 1 | G 1 | C 1 | C 1 | A 1 | A 1 | T 1 | T 1 | A 1 | A 1 | T 1 | T 1 | A 1 | A 1 | T 1 | T 1 | A 1 | A 1 | T 1 | T 1 | G 1 | G 1 | C 1 | C 1 | G 1 | G 1 | C 1 | C 1 |
| G 1 | G 1 | G 1 | G 1 | C 1 | C 1 | C 1 | C 1 | G 1 | G 1 | G 1 | G 1 | C 1 | C 1 | C 1 | C 1 | G 1 | G 1 | G 1 | G 1 | C 1 | C 1 | C 1 | C 1 | G 1 | G 1 | G 1 | G 1 | C 1 | C 1 | C 1 | C 1 | A 0 | A 0 | A 0 | A 0 | T 2 | T 2 | T 2 | T 2 |
| 3 | 3 | 3 | 3 | 3 | 3 | 3 | 3 | 2 | 4 | 2 | 4 | 2 | 4 | 2 | 4 | 2 | 2 | 4 | 4 | 2 | 2 | 4 | 4 | 1 | 3 | 3 | 5 | 1 | 3 | 3 | 5 | 2 | 2 | 2 | 2 | 4 | 4 | 4 | 4 |

| 2 | | | | | | | | 1 | | | | | | | | 0 | | | | | | | | 7 | 6 | 5 | 4 | 3 | 2 | 1 | 0 | Σ |
|---|---|---|---|---|---|---|---|---|---|---|---|---|---|---|---|---|---|---|---|---|---|---|---|---|---|---|---|---|---|---|---|---|
| A 1 | T 1 | A 1 | T 1 | A 1 | T 1 | A 1 | T 1 | G 1 | C 1 | G 1 | C 1 | G 1 | C 1 | G 1 | C 1 | A 1 | T 1 | A 1 | T 1 | A 1 | T 1 | A 1 | T 1 | 8 | 8 | 8 | 8 | 8 | 8 | 8 | 8 | **64** |
| G 1 | G 1 | C 1 | C 1 | G 1 | G 1 | C 1 | C 1 | A 1 | A 1 | T 1 | T 1 | A 1 | A 1 | T 1 | T 1 | A 1 | A 1 | T 1 | T 1 | A 1 | A 1 | T 1 | T 1 | 8 | 8 | 8 | 8 | 8 | 8 | 8 | 8 | **64** |
| A 0 | A 0 | A 0 | A 0 | T 2 | T 2 | T 2 | T 2 | A 0 | A 0 | A 0 | A 0 | T 2 | T 2 | T 2 | T 2 | A 0 | A 0 | A 0 | A 0 | T 2 | T 2 | T 2 | T 2 | 8 | 8 | 8 | 8 | 8 | 8 | 8 | 8 | **64** |
| 1 | 3 | 1 | 3 | 3 | 5 | 3 | 5 | 1 | 1 | 3 | 3 | 3 | 3 | 5 | 5 | 0 | 2 | 2 | 4 | 2 | 4 | 4 | 6 | **24** | **24** | **24** | **24** | **24** | **24** | **24** | **24** | **192** |

Fig. 12

| 7 | | | | | | | | 6 | | | | | | | | 5 | | | | | | | | 4 | | | | | | | | 3 | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|
| G<br>1 | C<br>1 | G<br>1 | C<br>1 | G<br>1 | C<br>1 | G<br>1 | C<br>1 | A<br>1 | U<br>1 | A<br>1 | U<br>1 | A<br>1 | U<br>1 | A<br>1 | U<br>1 | G<br>1 | C<br>1 | G<br>1 | C<br>1 | G<br>1 | C<br>1 | G<br>1 | C<br>1 | A<br>1 | U<br>1 | A<br>1 | U<br>1 | A<br>1 | U<br>1 | A<br>1 | U<br>1 | G<br>1 | C<br>1 | G<br>1 | C<br>1 | G<br>1 | C<br>1 | G<br>1 | C<br>1 |
| G<br>1 | G<br>1 | C<br>1 | C<br>1 | G<br>1 | G<br>1 | C<br>1 | C<br>1 | G<br>1 | G<br>1 | C<br>1 | C<br>1 | G<br>1 | G<br>1 | C<br>1 | C<br>1 | A<br>1 | A<br>1 | U<br>1 | U<br>1 | A<br>1 | A<br>1 | U<br>1 | U<br>1 | A<br>1 | A<br>1 | U<br>1 | U<br>1 | A<br>1 | A<br>1 | U<br>1 | U<br>1 | G<br>1 | G<br>1 | C<br>1 | C<br>1 | G<br>1 | G<br>1 | C<br>1 | C<br>1 |
| G<br>1 | G<br>1 | G<br>1 | G<br>1 | C<br>1 | C<br>1 | C<br>1 | C<br>1 | G<br>1 | G<br>1 | G<br>1 | G<br>1 | C<br>1 | C<br>1 | C<br>1 | C<br>1 | G<br>1 | G<br>1 | G<br>1 | G<br>1 | C<br>1 | C<br>1 | C<br>1 | C<br>1 | G<br>1 | G<br>1 | G<br>1 | G<br>1 | C<br>1 | C<br>1 | C<br>1 | C<br>1 | A<br>0 | A<br>0 | A<br>0 | A<br>0 | U<br>2 | U<br>2 | U<br>2 | U<br>2 |
| 3 | 3 | 3 | 3 | 3 | 3 | 3 | 3 | 2 | 4 | 2 | 4 | 2 | 4 | 2 | 4 | 2 | 2 | 4 | 4 | 2 | 2 | 4 | 4 | 1 | 3 | 3 | 5 | 1 | 3 | 3 | 5 | 2 | 2 | 2 | 2 | 4 | 4 | 4 | 4 |

| 2 | | | | | | | | 1 | | | | | | | | 0 | | | | | | | | 7 | 6 | 5 | 4 | 3 | 2 | 1 | 0 | Σ |
|---|---|---|---|---|---|---|---|---|---|---|---|---|---|---|---|---|---|---|---|---|---|---|---|---|---|---|---|---|---|---|---|---|
| A<br>1 | U<br>1 | A<br>1 | U<br>1 | A<br>1 | U<br>1 | A<br>1 | U<br>1 | G<br>1 | C<br>1 | G<br>1 | C<br>1 | G<br>1 | C<br>1 | G<br>1 | C<br>1 | A<br>1 | U<br>1 | A<br>1 | U<br>1 | A<br>1 | U<br>1 | A<br>1 | U<br>1 | 8 | 8 | 8 | 8 | 8 | 8 | 8 | 8 | **64** |
| G<br>1 | G<br>1 | C<br>1 | C<br>1 | G<br>1 | G<br>1 | C<br>1 | C<br>1 | A<br>1 | A<br>1 | U<br>1 | U<br>1 | A<br>1 | A<br>1 | U<br>1 | U<br>1 | A<br>1 | A<br>1 | U<br>1 | U<br>1 | A<br>1 | A<br>1 | U<br>1 | U<br>1 | 8 | 8 | 8 | 8 | 8 | 8 | 8 | 8 | **64** |
| A<br>0 | A<br>0 | A<br>0 | A<br>0 | U<br>2 | U<br>2 | U<br>2 | U<br>2 | A<br>0 | A<br>0 | A<br>0 | A<br>0 | U<br>2 | U<br>2 | U<br>2 | U<br>2 | A<br>0 | A<br>0 | A<br>0 | A<br>0 | U<br>2 | U<br>2 | U<br>2 | U<br>2 | 8 | 8 | 8 | 8 | 8 | 8 | 8 | 8 | **64** |
| 1 | 3 | 1 | 3 | 3 | 5 | 3 | 5 | 1 | 1 | 3 | 3 | 3 | 3 | 5 | 5 | 0 | 2 | 2 | 4 | 2 | 4 | 4 | 6 | **24** | **24** | **24** | **24** | **24** | **24** | **24** | **24** | **192** |

Fig. 13

| Parameter | Primary (major) | | | Minor (rare) | | |
|---|---|---|---|---|---|---|
| | (Adenine) Ade | (Guanine) Gua | Deviation | (Hypoxanthine) Hyp | (Xanthine) Xan | Deviation |
| Chemical formula | $C_5H_5N_5$ | $C_5H_5N_5O$ | O | $C_5H_4N_4O$ | $C_5H_4N_4O_2$ | O |
| Class | Purine | Purine | | Purine | Purine | |
| Number of atoms | 15 | 16 | 1 | 14 | 15 | 1 |
| Molar weight, gram-mol | 135.1272 | 151.1266 | 15.9994 | 136.1119 | 152.1114 | 15.9994 |
| Molar weight increment in the minor bases compared to the existing major bases, gram-mol | | | | 0.9847 | 0.9847 | 0.000000 |
| **Number of:** | | | | | | |
| electrons | 70 | 78 | 8 | 70 | 78 | 8 |
| protons | 70 | 78 | 8 | 70 | 78 | 8 |
| neutrons | 70 | 78 | 8 | 70 | 78 | 8 |
| Number of hydrogen bonds | 2 | 3 | 1 | 2 | 3 | 1 |
| Complimentary bases | T (U) | C | | T (U) | C | |
| Presence | DNA, RNA | DNA, RNA | | tRNA | tRNA | |
| Nucleoside = nitrogenous base + ribose (ribofuranose) residue | (Adenosine) Ado (A) | (Guanosine) Guo (G) | | (Inosine) Ino (I) | (Xanthosine) Xao (X) | |
| chemical formula | $C_{10}H_{13}N_5O_4$ | $C_{10}H_{13}N_5O_5$ | O | $C_{10}H_{12}N_4O_5$ | $C_{10}H_{12}N_4O_6$ | O |
| Molar weight increment in the minor nucleosides compared to the corresponding major bases, gram-mol | | | | 0.9847 | 0.9847 | 0.0000 |

Fig. 14

| | | | | | | | | | |
|---|---|---|---|---|---|---|---|---|---|
| pz | | 1 ez | 0 ez | 1 ez | 0 ez | 1 ez | 0 ez | 1 ez | 0 ez |
| py | | 1 ey | 1 ey | 0 ey | 0 ey | 1 ey | 1 ey | 0 ey | 0 ey |
| px | | 1 ex | 1 ex | 1 ex | 1 ex | 0 ex | 0 ex | 0 ex | 0 ex |
| hz | 1 | 1 1 0 | 0 1 1 | 1 1 0 | 0 1 1 | 1 1 0 | 0 1 1 | 1 1 0 | 0 1 1 |
| hy | 1 | 1 1 0 | 1 1 0 | 0 1 1 | 0 1 1 | 1 1 0 | 1 1 0 | 0 1 1 | 0 1 1 |
| hx | 1 | 1 1 0 | 1 1 0 | 1 1 0 | 1 1 0 | 0 1 1 | 0 1 1 | 0 1 1 | 0 1 1 |
| | | 1 1 0 | 0 1 1 | 0 1 1 | 1 1 0 | 0 1 1 | 1 1 0 | 1 1 0 | 0 1 1 |
| hz | 0 | 1 0 1 | 0 0 0 | 1 0 1 | 0 0 0 | 1 0 1 | 0 0 0 | 1 0 1 | 0 0 0 |
| hy | 1 | 1 1 0 | 1 1 0 | 0 1 1 | 0 1 1 | 1 1 0 | 1 1 0 | 0 1 1 | 0 1 1 |
| hx | 1 | 1 1 0 | 1 1 0 | 1 1 0 | 1 1 0 | 0 1 1 | 0 1 1 | 0 1 1 | 0 1 1 |
| | | 1 0 1 | 0 0 0 | 0 0 0 | 1 0 1 | 0 0 0 | 1 0 1 | 1 0 1 | 0 0 0 |
| hz | 1 | 1 1 0 | 0 1 1 | 1 1 0 | 0 1 1 | 1 1 0 | 0 1 1 | 1 1 0 | 0 1 1 |
| hy | 0 | 1 0 1 | 1 0 1 | 0 0 0 | 0 0 0 | 1 0 1 | 1 0 1 | 0 0 0 | 0 0 0 |
| hx | 1 | 1 1 0 | 1 1 0 | 1 1 0 | 1 1 0 | 0 1 1 | 0 1 1 | 0 1 1 | 0 1 1 |
| | | 1 0 1 | 0 0 0 | 0 0 0 | 1 0 1 | 0 0 0 | 1 0 1 | 1 0 1 | 0 0 0 |
| hz | 0 | 1 0 1 | 0 0 0 | 1 0 1 | 0 0 0 | 1 0 1 | 0 0 0 | 1 0 1 | 0 0 0 |
| hy | 0 | 1 0 1 | 1 0 1 | 0 0 0 | 0 0 0 | 1 0 1 | 1 0 1 | 0 0 0 | 0 0 0 |
| hx | 1 | 1 1 0 | 1 1 0 | 1 1 0 | 1 1 0 | 0 1 1 | 0 1 1 | 0 1 1 | 0 1 1 |
| | | 1 1 0 | 0 1 1 | 0 1 1 | 1 1 0 | 0 1 1 | 1 1 0 | 1 1 0 | 0 1 1 |
| hz | 1 | 1 1 0 | 0 1 1 | 1 1 0 | 0 1 1 | 1 1 0 | 0 1 1 | 1 1 0 | 0 1 1 |
| hy | 1 | 1 1 0 | 1 1 0 | 0 1 1 | 0 1 1 | 1 1 0 | 1 1 0 | 0 1 1 | 0 1 1 |
| hx | 0 | 1 0 1 | 1 0 1 | 1 0 1 | 1 0 1 | 0 0 0 | 0 0 0 | 0 0 0 | 0 0 0 |
| | | 1 0 1 | 0 0 0 | 0 0 0 | 1 0 1 | 0 0 0 | 1 0 1 | 1 0 1 | 0 0 0 |
| hz | 0 | 1 0 1 | 0 0 0 | 1 0 1 | 0 0 0 | 1 0 1 | 0 0 0 | 1 0 1 | 0 0 0 |
| hy | 1 | 1 1 0 | 1 1 0 | 0 1 1 | 0 1 1 | 1 1 0 | 1 1 0 | 0 1 1 | 0 1 1 |
| hx | 0 | 1 0 1 | 1 0 1 | 1 0 1 | 1 0 1 | 0 0 0 | 0 0 0 | 0 0 0 | 0 0 0 |
| | | 1 1 0 | 0 1 1 | 0 1 1 | 1 1 0 | 0 1 1 | 1 1 0 | 1 1 0 | 0 1 1 |
| hz | 1 | 1 1 0 | 0 1 1 | 1 1 0 | 0 1 1 | 1 1 0 | 0 1 1 | 1 1 0 | 0 1 1 |
| hy | 0 | 1 0 1 | 1 0 1 | 0 0 0 | 0 0 0 | 1 0 1 | 1 0 1 | 0 0 0 | 0 0 0 |
| hx | 0 | 1 0 1 | 1 0 1 | 1 0 1 | 1 0 1 | 0 0 0 | 0 0 0 | 0 0 0 | 0 0 0 |
| | | 1 1 0 | 0 1 1 | 0 1 1 | 1 1 0 | 0 1 1 | 1 1 0 | 1 1 0 | 0 1 1 |
| hz | 0 | 1 0 1 | 0 0 0 | 1 0 1 | 0 0 0 | 1 0 1 | 0 0 0 | 1 0 1 | 0 0 0 |
| hy | 0 | 1 0 1 | 1 0 1 | 0 0 0 | 0 0 0 | 1 0 1 | 1 0 1 | 0 0 0 | 0 0 0 |
| hx | 0 | 1 0 1 | 1 0 1 | 1 0 1 | 1 0 1 | 0 0 0 | 0 0 0 | 0 0 0 | 0 0 0 |
| | | 1 0 1 | 0 0 0 | 0 0 0 | 1 0 1 | 0 0 0 | 1 0 1 | 1 0 1 | 0 0 0 |

Fig. 15

| | 7 | 6 | 5 | 4 | 3 | 2 | 1 | 0 |
|---|---|---|---|---|---|---|---|---|
| | h 0 0 0 | 0 0 1 | 0 1 0 | 0 1 1 | 1 0 0 | 1 0 1 | 1 1 0 | 1 1 1 |
| **7** | p 1 1 0 | 0 1 1 | 0 1 1 | 1 1 0 | 0 1 1 | 1 1 0 | 1 1 0 | 0 1 1 |
| | **A A T** | **T A G** | **T G A** | **A G C** | **C A A** | **G A C** | **G G T** | **C G G** |
| | h 0 0 1 | 0 0 0 | 0 1 1 | 0 1 0 | 1 0 1 | 1 0 0 | 1 1 1 | 1 1 0 |
| **6** | p 1 0 1 | 0 0 0 | 0 0 0 | 1 0 1 | 0 0 0 | 1 0 1 | 1 0 1 | 0 0 0 |
| | **A T G** | **T T T** | **T C C** | **A C A** | **C T C** | **G T A** | **G C G** | **C C T** |
| | h 0 1 0 | 0 1 1 | 0 0 0 | 0 0 1 | 1 1 0 | 1 1 1 | 1 0 0 | 1 0 1 |
| **5** | p 1 0 1 | 0 0 0 | 0 0 0 | 1 0 1 | 0 0 0 | 1 0 1 | 1 0 1 | 0 0 0 |
| | **A C A** | **T C C** | **T T T** | **A T G** | **C C T** | **G C G** | **G T A** | **C T C** |
| | h 0 1 1 | 0 1 0 | 0 0 1 | 0 0 0 | 1 1 1 | 1 1 0 | 1 0 1 | 1 0 0 |
| **4** | p 1 1 0 | 0 1 1 | 0 1 1 | 1 1 0 | 0 1 1 | 1 1 0 | 1 1 0 | 0 1 1 |
| | **A G C** | **T G A** | **T A G** | **A A T** | **C G G** | **G G T** | **G A C** | **C A A** |
| | h 1 0 0 | 1 0 1 | 1 1 0 | 1 1 1 | 0 0 0 | 0 0 1 | 0 1 0 | 0 1 1 |
| **3** | p 1 0 1 | 0 0 0 | 0 0 0 | 1 0 1 | 0 0 0 | 1 0 1 | 1 0 1 | 0 0 0 |
| | **G T A** | **C T C** | **C C T** | **G C G** | **T T T** | **A T G** | **A C A** | **T C C** |
| | h 1 0 1 | 1 0 0 | 1 1 1 | 1 1 0 | 0 0 1 | 0 0 0 | 0 1 1 | 0 1 0 |
| **2** | p 1 1 0 | 0 1 1 | 0 1 1 | 1 1 0 | 0 1 1 | 1 1 0 | 1 1 0 | 0 1 1 |
| | **G A C** | **C A A** | **C G G** | **G G T** | **T A G** | **A A T** | **A G C** | **T G A** |
| | h 1 1 0 | 1 1 1 | 1 0 0 | 1 0 1 | 0 1 0 | 0 1 1 | 0 0 0 | 0 0 1 |
| **1** | p 1 1 0 | 0 1 1 | 0 1 1 | 1 1 0 | 0 1 1 | 1 1 0 | 1 1 0 | 0 1 1 |
| | **G G T** | **C G G** | **C A A** | **G A C** | **T G A** | **A G C** | **A A T** | **T A G** |
| | h 1 1 1 | 1 1 0 | 1 0 1 | 1 0 0 | 0 1 1 | 0 1 0 | 0 0 1 | 0 0 0 |
| **0** | p 1 0 1 | 0 0 0 | 0 0 0 | 1 0 1 | 0 0 0 | 1 0 1 | 1 0 1 | 0 0 0 |
| | **G C G** | **C C T** | **C T C** | **G T A** | **T C C** | **A C A** | **A T G** | **T T T** |

Fig. 16

Table

| Decimal triplet code | Triplet for DNA | Triplet for RNA | Amino acid | Indexing information | Latin alphabet | Russian alphabet |
|---|---|---|---|---|---|---|
| 63 | *GGG* | *GGG* | Gly (G) | 111111 | space | |
| 62 | *GGC* | *GGC* | Gly (G) | 111110 | capital letter | |
| 61 | *GCG* | *GCG* | Ala (A) | 111101 | | Ж |
| 60 | *GCC* | *GCC* | Ala (A) | 111100 | . (dot) | |
| 59 | *CGG* | *CGG* | Arg (R) | 111011 | , (comma) | |
| 58 | *CGC* | *CGC* | Arg (R) | 111010 | ; (semicolon) | |
| 57 | *CCG* | *CCG* | Pro (P) | 111001 | | З |
| 56 | *CCC* | *CCC* | Pro (P) | 111000 | | Я |
| 55 | *GGA* | *GGA* | Gly (G) | 110111 | | Щ |
| 54 | *GGT* | *GGU* | Gly (G) | 110110 | | Ш |
| 53 | *GCA* | *GCA* | Ala (A) | 110101 | enter; new paragraph; = | |
| 52 | *GCT* | *GCU* | Ala (A) | 110100 | | Ю |
| 51 | *CGA* | *CGA* | Arg (R) | 110011 | | Ы |
| 50 | *CGT* | *CGU* | Arg (R) | 110010 | | Ъ |
| 49 | *CCA* | *CCA* | Pro (P) | 110001 | | Ь |
| 48 | *CCT* | *CCU* | Pro (P) | 110000 | ? (question mark) | |
| 47 | *GAG* | *GAG* | Glu (E) | 101111 | | П |
| 46 | *GAC* | *GAC* | Asp (D) | 101110 | | Э |
| 45 | *GTG* | *GUG* | Val (V) | 101101 | number | |
| 44 | *GTC* | *GUC* | Val (V) | 101100 | | Ч |
| 43 | *CAG* | *CAG* | Gln (Q) | 101011 | | Л |
| 42 | *CAC* | *CAC* | His (H) | 101010 | | Ц |
| 41 | *CTG* | *CUG* | Leu (L) | 101001 | | Й |
| 40 | *CTC* | *CUC* | Leu (L) | 101000 | | И |
| 39 | *GAA* | *GAA* | Glu (E) | 100111 | «» (quotation marks) | |
| 38 | *GAT* | *GAU* | Asp (D) | 100110 | | Г |
| 37 | *GTA* | *GUA* | Val (V) | 100101 | | Ф |
| 36 | *GTT* | *GUU* | Val (V) | 100100 | | Ё |
| 35 | *CAA* | *CAA* | Gln (Q) | 100011 | | Д |
| 34 | *CAT* | *CAU* | His (H) | 100010 | color | |
| 33 | *CTA* | *CUA* | Leu (L) | 100001 | ( ) (parentheses) | |
| 32 | *CTT* | *CUU* | Leu (L) | 100000 | | Б |
| 31 | *AGG* | *AGG* | Arg (R) | 011111 | – (minus/ dash) | |
| 30 | *AGC* | *AGC* | Ser (S) | 011110 | + (plus) | |

Table continues

| Decimal triplet code | Triplet for DNA | Triplet for RNA | Amino acid | Indexing information | Latin alphabet | Russian alphabet |
|---|---|---|---|---|---|---|
| 29 | *ACG* | *ACG* | *Thr (T)* | 011101 | ∗ (multiplication) | |
| 28 | *ACC* | *ACC* | *Thr (T)* | 011100 | ^ (raising to power) | |
| 27 | *TGG* | *UGG* | *Trp (W)* | 011011 | / (÷) (division) | |
| 26 | *TGC* | *UGC* | *Cys (C)* | 011010 | ! (factorial) | |
| 25 | *TCG* | *UCG* | *Ser (S)* | 011001 | Z | |
| 24 | *TCC* | *UCC* | *Ser (S)* | 011000 | y | y |
| 23 | *AGA* | *AGA* | *Arg (R)* | 010111 | X | X |
| 22 | *AGT* | *AGU* | *Ser (S)* | 010110 | W | |
| 21 | *ACA* | *ACA* | *Thr (T)* | 010101 | V | |
| 20 | *ACT* | *ACU* | *Thr (T)* | 010100 | U | |
| 19 | *TGA* | *UGA* | *Opal* | 010011 | T | T |
| 18 | *TGT* | *UGU* | *Cys (C)* | 010010 | S | |
| 17 | *TCA* | *UCA* | *Ser (S)* | 010001 | R | |
| 16 | *TCT* | *UCU* | *Ser (S)* | 010000 | Q | |
| 15 | *AAG* | *AAG* | *Lys (K)* | 001111 | P | P |
| 14 | *AAC* | *AAC* | *Asn (N)* | 001110 | O | O |
| 13 | *ATG* | *AUG* | *Met (M)* | 001101 | N | |
| 12 | *ATC* | *AUC* | *Ile (I)* | 001100 | M | M |
| 11 | *TAG* | *UAG* | *Amber (янтарь)* | 001011 | L | |
| 10 | *TAC* | *UAC* | *Tyr ( Y)* | 001010 | K | K |
| 9 | *TTG* | *UUG* | *Leu (L)* | 001001 | J | |
| 8 | *TTC* | *UUC* | *Phe (F)* | 001000 | I | |
| 7 | *AAA* | *AAA* | *Lys (K)* | 000111 | H | H |
| 6 | *AAT* | *AAU* | *Asn (N)* | 000110 | G | |
| 5 | *ATA* | *AUA* | *Ile (I)* | 000101 | F | |
| 4 | *ATT* | *AUU* | *Ile (I)* | 000100 | E | E |
| 3 | *TAA* | *UAA* | *Ochre (охра)* | 000011 | D | |
| 2 | *TAT* | *UAU* | *Tyr ( Y)* | 000010 | C | C |
| 1 | *TTA* | *UUA* | *Leu (L)* | 000001 | B | B |
| 0 | *TTT* | *UUU* | *Phe (F)* | 000000 | A | A |

Fig. 17

Europäisches Patentamt
European Patent Office
Office européen des brevets

# EUROPEAN SEARCH REPORT

Application Number
EP 18 17 7823

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 512 749 A2 (SONY CORP [JP]) 9 March 2005 (2005-03-09) * [0076]-[0078], [0148]-[0152], [0167]-[0182], [0242]-[0249] * | 1-20 | INV. G06F19/22 B82Y10/00 |
| X | LEONID V. BYSTRYKH: "Generalized DNA Barcode Design Based on Hamming Codes", PLOS ONE, vol. 7, no. 5, 17 May 2012 (2012-05-17), page e36852, XP055374134, DOI: 10.1371/journal.pone.0036852 * pgs. 2-5, Tables 1-3, Fig. 1 * | 1-20 | |
| X | WO 2013/178801 A2 (EUROPEAN MOLECULAR BIOLOGY LAB EMBL [DE]) 5 December 2013 (2013-12-05) * [0045],[0060]-[0073] * | 1-20 | |
| X | WO 2015/144858 A1 (THOMSON LICENSING [FR]) 1 October 2015 (2015-10-01) * abstract, pgs. 2-9 * | 1-20 | TECHNICAL FIELDS SEARCHED (IPC) |
| X | TILO BUSCHMANN ET AL: "Levenshtein error-correcting barcodes for multiplexed DNA sequencing", BMC BIOINFORMATICS, vol. 14, no. 1, 1 January 2013 (2013-01-01), page 272, XP055194297, ISSN: 1471-2105, DOI: 10.1186/1471-2105-14-272 * pgs. 2-4 * | 1-20 | G06F |
| X | US 2010/323348 A1 (HAMADY MICAH L [US] ET AL) 23 December 2010 (2010-12-23) * abstract, [0079]-[0110] * | 1-20 | |
| | -/-- | | |

The present search report has been drawn up for all claims

2

| Place of search | Date of completion of the search | Examiner |
|---|---|---|
| Munich | 6 November 2018 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 17 7823

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/042372 A1 (ARITA MASANORI [JP]) 22 February 2007 (2007-02-22) * abstract, Fig. 1, [0031]-[0035] * ----- | 1-20 | |
| X | DAN TULPAN ET AL: "HyDEn: A Hybrid Steganocryptographic Approach for Data Encryption Using Randomized Error-Correcting DNA Codes", BIOMED RESEARCH INTERNATIONAL, vol. 32, no. 4839, 1 January 2013 (2013-01-01), pages 675-11, XP055194313, ISSN: 2314-6133, DOI: 10.1155/2013/634832 * abstract, pgs. 3-5 * ----- | 1-20 | |
| | | | TECHNICAL FIELDS SEARCHED (IPC) |

The present search report has been drawn up for all claims

| Place of search | Date of completion of the search | Examiner |
|---|---|---|
| Munich | 6 November 2018 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

page 2 of 2

# ANNEX TO THE EUROPEAN SEARCH REPORT ON EUROPEAN PATENT APPLICATION NO. EP 18 17 7823

This annex lists the patent family members relating to the patent documents cited in the above-mentioned European search report. The members are as contained in the European Patent Office EDP file on
The European Patent Office is in no way liable for these particulars which are merely given for the purpose of information.

06-11-2018

| Patent document cited in search report | Publication date | Patent family member(s) | Publication date |
|---|---|---|---|
| EP 1512749 A2 | 09-03-2005 | EP 1512749 A2 | 09-03-2005 |
| | | JP 2005080523 A | 31-03-2005 |
| | | US 2005108781 A1 | 19-05-2005 |
| WO 2013178801 A2 | 05-12-2013 | AU 2013269536 A1 | 18-12-2014 |
| | | CA 2874540 A1 | 05-12-2013 |
| | | CN 104520864 A | 15-04-2015 |
| | | CN 107055468 A | 18-08-2017 |
| | | EP 2856375 A2 | 08-04-2015 |
| | | EP 3346404 A1 | 11-07-2018 |
| | | HK 1208937 A1 | 18-03-2016 |
| | | JP 2015529864 A | 08-10-2015 |
| | | KR 20150016572 A | 12-02-2015 |
| | | RU 2014152796 A | 27-07-2016 |
| | | SG 11201407818P A | 30-12-2014 |
| | | US 2015261664 A1 | 17-09-2015 |
| | | WO 2013178801 A2 | 05-12-2013 |
| WO 2015144858 A1 | 01-10-2015 | EP 3123376 A1 | 01-02-2017 |
| | | US 2017187390 A1 | 29-06-2017 |
| | | WO 2015144858 A1 | 01-10-2015 |
| US 2010323348 A1 | 23-12-2010 | NONE | |
| US 2007042372 A1 | 22-02-2007 | CN 1791875 A | 21-06-2006 |
| | | JP 2004355294 A | 16-12-2004 |
| | | US 2007042372 A1 | 22-02-2007 |
| | | WO 2004107243 A1 | 09-12-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6312911 B
- US 20050053968 A
- RU 2408979
- US 61654295 B


### Non-patent literature cited in the description


- **I.V. AGAFONOVA.** *Cryptographic Properties of Non-Linear Boolean Functions,* 2007, http://dha.spb.ru/PDF/cryptoBOOLEAN.pdf
- **AREFYEV, V.A. ; LISOVENKO, L.A.** *English-Russian Dictionary of Genetic Terms,* 1995, 407